# EUROPEAN PATENT SPECIFICATION

(11) **EP 2 020 225 B1**
(45) Date of publication and mention of the grant of the patent: **08.10.2014**
(21) Application number: 08166557.2
(22) Date of filing: 08.08.2003
(51) Int. Cl.: A61K 8/37, A61Q 5/00, A61Q 13/00

(54) **Fragrance composition**
Duftstoffzusammensetzung
Composition aromatique

(30) Priority: 09.08.2002 JP 2002234104
(43) Date of publication of application: 04.02.2009
(62) Divisional of application: 03784610.2
(73) Proprietor: KAO CORPORATION, Tokyo 103-8210 (JP)
(72) Inventor: Ishihara, Hideki, Tokyo 131-8501 (JP); Fujihara, Hirohisa, Tokyo 131-8501 (JP); Takada, Yoshihiro, Tokyo 131-8501 (JP); Terazaki, Hiroyuki, Tokyo 131-8501 (JP)
(74) Representative: HOFFMANN EITLE

(56) References cited:
- US-A- 5 540 853
- US-A1- 2003 066 141

## Description

### Field of the Invention

This invention relates to fragrance compositions which exhibit a high masking effect on acid smell, retain good long-term stability at high temperatures, give off a good scent from a bottle in which the hair cosmetic composition is filled as well as a good scent upon application of the hair cosmetic composition and are excellent in the persistence of such scent after the application, even when added to a hair cosmetic composition having a pH of 1 to 5.

### Background of the Invention

Conventional hair cosmetic compositions generally have a pH in the neutral range and contain little acid therein, so no problem has been manifested about an acid smell from a cosmetic base. Further, no attention has been paid to fragrances added to hair cosmetic preparations or their stability in the acidic range.

With the consumers' needs changing in recent years, developments have been made to formulate hair cosmetic compositions having a pH in the acidic range such that various functions can be imparted to them. Acidic hair cosmetic compositions, however, give off a peculiar acid smell. Aneed has, therefore, arisen to mask a smell of a cosmetic base contained in acidic hair cosmetic compositions.

Acidic hair cosmetic compositions are low in pH. When fragrances added to conventional hair cosmetic compositions are simply added to such acidic hair cosmetic compositions, they develop such a problem that an odor balance deteriorates and an unpleasant odor manifests (JP-A-2000-143453).

US 5 540 853 A (TRINH TOAN [US] ET AL) 30 July 1996 (1996-07-30) discloses (ex. 40-43) a hair cosmetic composition having a pH of 4.5, comprising:
(I) 1-50% surfactant: Na Mg laureth sulfate, lauroamphoacetate etc...
(II) 0.05-10 % oil: soybean oil
(III) 0.05-10 % organic acid: myristic/citric acid
(IV) a fragrance composition comprising:
   - lilial
   - hexyl cinnamic aldehyde, benzyl acetate
   - coumarin, methyl ionone.

The present invention provides a hair cosmetic composition comprising a fragrance composition which can mask a smell peculiar to an acidic hair cosmetic composition and is also excellent in long-term stability. The fragrance composition gives off a good scent from a bottle in which a hair cosmetic composition is filled, as well as a good scent upon application of the hair cosmetic composition, and is excellent in the persistence of such scent after the application.

### Disclosure of the Invention

For a fragrance composition to be added to an acidic hair cosmetic composition having a pH of 1 to 5, on the other hand, it is important as basic requirements to mask an acid smell derived from a cosmetic base and to have good long-term storage stability. Further, from the viewpoint of its comfortable use by consumers and the enhancement of its attraction as merchandise, it is also important that the hair cosmetic composition has a good scent at each of the following three stages:
1. The scent emitted from the bottle mouth is favorable since this serves as one of the important factors for consumers upon selecting a brand at the stores.
2. The scent upon its application is favorable.
3. A favorable scent remains at an adequate intensity on the hair after the application.

The present invention provides a hair cosmetic composition having a pH of 1 to 5, according to claim 1.

### Detailed Description of the Invention

As the musk employed as ingredient (A) in the fragrance composition according to the present invention, a synthetic musk can be mentioned. Specific examples include muscone, civetone, cyclopentadecanone, 5-cyclohexadecen-1-one, cyclopentadecanolide, ambrettolide, 12-ketocyclopentadecanolide, cyclohexadecanolide, 7-cyclohexadecanolide, 12-oxa-16-hexadecanolide, 11-oxa-16-hexadecanolide, 10-oxa-16-hexadecanolide, ethylene brassylate, 3-methycyclopentadecenone (muscenone, NF), cyclopentadenolide (pentalide), ethylenedodecane dioate, musk ketone, 6-acetylhexamethylindan ("PHANTOLID", trade name of PFW Aroma Chemicals B.V.), 4-acetyldimethyl-t-butylindan ("CELESTOLIDE", trade name of International Flavors & Fragrances Inc.), 5-acetyltetramethylisopropylindan ("TRASEOLIDE", trade name of Quest International B.V.), 6-acetylhexatetralin ("TENTAROME", trade name of PFW Aroma Chemicals B.V.), tetramethyl-6-ethyl-7-acetyl-tetrahydronaphthalene ("VERSALIDE", trade name of Givaudan-Roure Corporation), formylethyltetramethyltetralin, acetyldimethyltetrahydrobenzindanone ("VITALIDE", trade name of Takasago International Corporation), hexamethylhexahydrocyclopentabenzopyran ("GALAXOLIDE", trade name of International Flavors & Fragrances Inc.), and 3-methylcyclopentadeceone ("MUSCENONE DELTA", Firmerich, Inc.). Among these synthetic musks, preferred examples are muscone, ambrettolide, ethylene brassylate, musk ketone, 3-methylcyclopentadecenone (muscenone, NF), cyclopentadecenolide (pentalide), hexamethylhexahydrocyclopentabenzopyran ("GALAXOLIDE", trade name of International Flavors & Fragrances Inc.), and 3-methylcyclopentadeceone ("MUSCENONE DELTA", Firmenich, Inc.).

From the viewpoint of including an amount sufficient to mask an acid smell, ensuring a harmony in fragrance with other materials and imparting mildness to fragrance, the content of the musk is from 0.1 to 70 wt.%, preferably from 1 to 50 wt.%, more preferably from 2 to 40 wt.% of the fragrance composition.

Ingredient (B) employed in the present invention consists of one or more compounds selected from the above-described compounds (i) to (v), although a combination of two or more compounds is preferred.

In ingredient (B)(i) of formula (1), the groups represented by R¹ and R² can each be a linear, branched or cyclic hydrocarbon group, or a group containing a linear, branched or cyclic hydrocarbon group with an oxygen atom or nitrogen atom inserted in at least one carbon-to-carbon bond thereof. It is to be noted that the term "hydrocarbon group" as used herein includes both saturated and unsaturated ones and the term "cyclic hydrocarbon group" as used herein includes saturated, unsaturated and aromatic, cyclic hydrocarbon groups. As the atom inserted in the at least one carbon-to-carbon bond, an oxygen atom or a nitrogen atom can bementioned, with an oxygen atombeingpreferred. A preferred form of linkage with an oxygen atom contained therein is an ether linkage in a linear ether or cyclic ether. R¹ has from 2 to 14 carbon atoms, while R² has from 1 to 15 carbon atoms.

Preferred examples of R¹ and R² include alkyl groups, alkenyl groups, cyclic hydrocarbon groups, cyclic hydrocarbyl-alkyl groups, cyclic hydrocarbyl-alkenyl groups, aromatic hydrocarbon groups, aromatic hydrocarbyl-alkyl groups, aromatic hydrocarbyl-alkenyl groups, and monoterpene and other terpene groups.

Examples of the compound of formula (1) include terpenyl esters, aliphatic esters, and aromatic esters. Illustrative of the terpenyl esters of formula (1) are citronellyl propionate, geranylpropionate, linalylpropionate, terpinyl propionate, rhodinyl propionate, neryl propionate, carbinyl propionate, menthyl propionate, bornyl propionate, isobornyl propionate, linalyl butyrate, geranyl butyrate, citronellyl butyrate, rhodinyl butyrate, neryl butyrate, terpenyl butyrate, santalyl butyrate, citronellyl isobutyrate, geranyl isobutyrate, linalyl isobutyrate, rhodinyl isobutyrate, neryl isobutyrate, terpinyl isobutyrate, linalyl isovalerate, citronellyl isovalerate, geranyl isovalerate, menthyl isovalerate, terpinyl isovalerate, linalyl hexanoate, citronellyl hexanoate, geranyl hexanoate, linalyl octanoate, citronellyl tiglate, geranyl benzoate, linalyl benzoate, geranyl phenylacetate, citronellyl phenylacetate, rhodinylphenylacetate, menthylphenylacetate, linalyl cinnamate, citronellyl tiglate, geranyl tiglate, methyl geranate, ethyl geranate, methyl cyclogeranate, ethyl cyclogeranate, and ethylcitronellyl oxalate.

Examples of the aliphatic esters of formula (1) include ethyl propionate, propyl propionate, allyl propionate, butyl propionate, isobutyl propionate, isoamyl propionate, hexyl propionate, cis-3-hexenyl propionate, trans-2-hexenyl propionate, decenyl propionate, tricyclodecenyl propionate, methyl butyrate, ethyl butyrate, propyl butyrate, isopropyl butyrate, allyl butyrate, butyl butyrate, isobutyl butyrate, amyl butyrate, isoamyl butyrate, hexyl butyrate, heptyl butyrate, cis-3-hexenyl butyrate, trans-2-hexenyl butyrate, octyl butyrate, propylene glycol dibutyrate, cyclohexyl butyrate, tetrahydrofurfuryl butyrate, methyl isobutyrate, ethyl isobutyrate, propyl isobutyrate, isopropyl isobutyrate, butyl isobutyrate, isobutyl isobutyrate, isoamyl isobutyrate, hexyl isobutyrate, cis-3-hexenylisobutyrate, 2, 4-hexadienyl isobutyrate, 1,3-dimethyl-3-butenyl isobutyrate, octyl isobutyrate, tricyclodecenyl isobutyrate, methyl 2-methylbutyrate, ethyl 2-methylbutyrate, 2-methylbutyl 2-methylbutyrate, hexyl 2-metylbutyrate, cis-3-hexenyl 2-methylbutyrate, allyl 2-ethylbutyrate, ethyl 3-hydroxybutyrate, methyl valerate, ethyl valerate, propyl valerate, butyl valerate, isobutyl valerate, amyl valerate, cis-3-hexenyl valerate, methyl isovalerate, ethyl isovalerate, propyl isovalerate, isopropyl isovalerate, allyl isovalerate, butyl isovalerate, isobutyl isovalerate, isoamyl isovalerate, 2-methylbutyl isovalerate, hexyl isovalerate, heptyl isovalerate, ethyl tricyclo[5.2.1.0^{2,6}]decan-2-yl carboxylate ("FRUITATE", Kao Corporation), and ethyl 2-cyclohexylpropionate ("POIRENATE", Kao Corporation). Preferred are butyl propionate, ethyl butyrate, propyl butyrate, isopropyl butyrate, butyl butyrate, isobutyl butyrate, amyl butyrate, isoamyl butyrate, cis-3-hexenyl butyrate, ethyl isobutyrate, butyl isobutyrate, isobutyl isobutyrate, ethyl 2-methylbulyrate, 2-methylbutyl 2-methylbutyrate, ethyl valerate, butyl valerate, isobutyl valerate, amyl valerate, ethyl isovalerate, butyl isovalerate, isobutyl isovalerate, isoamyl isovalerate, 2-methylbutyl isovalerate, ethyl tricyclo[5.2.1.0^{2,6}]decan-2-yl carboxylate ("FRUITATE", Kao Corporation), and ethyl 2-cyclohexylpropionate ("POIRENATE", Kao Corporation).

Examples of the aromatic esters of formula (1) include ethyl benzylacetoacetate, benzyl propionate, styralyl propionate, anisyl propionate, phenylethyl propionate, cinnamyl propionate, phenylpropyl propionate, dimethylbenzylcarvinyl propionate, phenoxyethyl propionate, propylene glycol dipropionate, ethyl 3-hydroxy-3-phenylpropionate, isobutyl furanpropionate, benzyl butyrate, cinnamyl butyrate, phenylethyl butyrate, dimethylbenzylcarvinyl butyrate, benzyl isobutyrate, p-cresyl isobutyrate, cinnamyl isobutyrate, phenylethyl isobutyrate, phenoxyethyl isobutyrate, phenylpropyl isobutyrate, styrallyl isobutyrate, dimethylbenzylcarvinyl isobutyrate, dimethylpheylethylcarvinyl isobutyrate, decahydro-β-naphthyl isobutyrate, benzyl 2-methylbutyrate, phenylethyl 2-methylbutyrate, benzyl valerate, phenylethyl valerate, furfuryl valerate, benzyl isobutyrate, cinnamyl isovalerate, phenylethyl isovalerate, phenylpropyl isovalerate, benzyl hexanoate, benzyl octanoate, phenylethyl octanoate, p-cresyl octanoate, phenylethyl nonaoate, benzyl dodecanoate(benzyl laurate), methyl benzoate, ethylbenzoate, propylbenzoate, isopropylbenzoate, allylbenzoate, isobutyl benzoate, isoamyl benzoate, prenyl benzoate, hexyl benzoate, cis-3-hexenyl benzoate, benzyl benzoate, phenylethyl benzoate, ethyl o-methoxybenzoate, methyl dihydroxydimethylbenzoate, methyl phenylacetate, ethyl phenylacetate, propylphenylacetate, isopropylphenylacetate, butyl phenylacetate, isobutyl phenylacetate, isoamyl phenylacetate, hexyl phenylacetate, cis-3-hexenyl phenylacetate, benzyl phenylacetate, phenylethyl phenylacetate, p-cresylphenylacetate, eugenylphenylacetate, isoeugenyl phenylacetate, methyl cinnamate, ethyl cinnamate, propylcinnamate, isopropyl cinnamate, allyl cinnamate, isobutyl cinnamate, isoamyl cinnamate, benzyl cinnamate, cinnamyl cinnamate, Phenylethyl cinnamate, dimethyl phthalate, diethyl phthalate, methyl salicylate, ethyl salicylate, butyl salicylate, isobutyl salicylate, amyl salicylate, isoamyl salicylate, allyl salicylate, hexyl salicylate, cis-3-hexenyl salicylate, cyclohexyl salicylate, phenyl salicylate, benzylsalicylate, phenylethyl salicylate, p-cresyl salicylate, allyl phenoxyacetate, ethyl phenylpropionate, benzyl tiglate, phenylethyl tiglate, cinnamyl tiglate, benzyl angelate, phenylethyl angelate, cinnamyl angelate, and phenyl angelate.

Preferred are benzyl isovalerate, cinnamyl isovalerate, phenylethyl isovalerate, ethyl benzoate, propyl benzoate, isopropyl benzoate, allyl benzoate, isobutyl benzoate, isoamyl benzoate, prenyl benzoate, hexyl benzoate, cis-3-hexenyl benzoate, benzyl benzoate, phenylethyl benzoate, methyl cinnamate, ethyl cinnamate, methyl salicylate, ethyl salicylate, amyl salicylate, isoamyl salicylate, hexyl salicylate, and cis-3-hexenyl salicylate. The content of ingredient (B) (i) is from 0.001 to 80 wt.%, preferably from 1 to 80 wt.%, more preferably from 1.5 to 60 wt.% of the fragrance composition.

In ingredient (B)(ii) of formula (2), the hydrocarbon group or the cyclic hydrocarbon group represented by R⁴, in which an α-carbon or β-carbon to an ether linkage in an ester group in formula (2) has a branched chain, can be a group represented by the following formula (2a) or (2b): 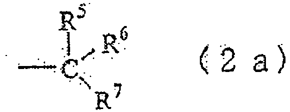 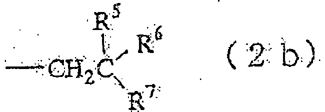 wherein R⁵ represents a hydrogen atom or an alkyl or alkenyl group having from 1 to 14 carbon atoms or forms an unsaturated bond with a carbon atom in R⁶ or R⁷, R⁶ and R⁷ each represents an alkyl or alkenyl group having 1 to 14 carbon atoms or R⁵ and R⁶ are fused together to form a saturated or unsaturated, cyclic hydrocarbon group having 4 to 8 carbon atoms, said cyclic hydrocarbon group being optionally substituted by one or more alkyl or alkenyl groups.

Examples of the compound of formula (2) include terpenyl esters of formic acid and acetic acid, fatty esters of formic acid and acetic acid, and aromatic esters of formic acid and acetic acid. Illustrative of the terpenyl esters of formic acid and acetic acid are linalyl formate, citronellyl formate, geranyl formate, neryl formate, rhodinyl formate, terpinyl formate, cedryl formate, caryophyllene formate, ocimenyl acetate, citronellyl acetate, lavandulyl acetate, isodihydrolavandulyl acetate, nerolidol acetate, geranyl acetate, linalyl acetate, myrcenyl acetate, dihydromyrcenyl acetate, rhodinyl acetate, neryl acetate, tetrahydromugol acetate, ethyllinalyl acetate, carvyl acetate, dihydrocarvyl acetate, dihydrocuminyl acetate, terpinyl acetate, dihydrocarbinyl acetate, isopregol acetate, menthyl acetate, dihydroterpinyl acetate (menthanyl acetate), citryl acetate, myrcenyl acetate, nopyl acetate, penchyl acetate, n-bornyl acetate, isobornyl acetate, guaiyl acetate, cedryl acetate, verbenyl acetate, caryophyllene acetate, santalyl acetate, vetiveryl acetate, and guaiac acetate. Preferred is linalyl acetate.

Examples of the aliphatic esters of formic acid and acetic acid include "APHERMATE" (trade name of International Flavors & Fragrances Inc.), oxyoctaline formate, isopropyl acetate, isobutyl acetate, 3-octyl acetate, cyclohexyl acetate, p-t-butylcyclohexyl acetate, 2,4-dimethyl-3-cyclohexenylmethyl acetate, α,3,3,-trimethylcyclohexanemethyl acetate ("ROSAMUSK", trade name of International Flavors & Fragrances Inc.), o-t-butylcyclohexyl acetate, 1-ethylcyclohexyl acetate, tricyclodecenyl acetate, 2,4-dimethyl-cyclohexen-1-methanyl acetate ("FLORALATE", trade name of International Flavors & Fragrances Inc.), decahydro-β-naphthyl acetate, 1-acetoxy-2-sec-butyl-1-vinylcyclohexane, tricyclodecyl acetate, tetrahydrofurfuryl acetate, 3-pentyltetrahydropyranyl acetate ("JASMAL", trade name of International Flavors & Fragrances Inc.), 5-methyl-3-butyltetrahydropyranyl acetate ("JASMELIA", trade name of International Flavors & Fragrances Inc.), ethyl acetoacetate, ethyl 2-hexylacetoacetate, methyl cyclopentylidenacetate, allyl cyclohexylacetate, isopropyl cyclohexenylacetate, and o-t-butylcyclohexyl acetate. Preferred are tricyclodecenyl acetate and o-t-butylcyclohexyl acetate.

Examples of the aromatic esters of formic acid and acetic acid include benzyl formate, methylphenylcarbinyl acetate, styralyl acetate, p-methylbenzyl acetate, anisyl acetate, piperonyl acetate, acetyl vanillin, rosephenone, hydratropyl acetate, 2,4-dimethylbenzyl acetate, cuminyl acetate, dimethylbenzylcarbinyl acetate, heliotropyl acetate, eugenol acetate, isoeugenol acetate, phenylglycol diacetate, dimethylphenylcarbinyl acetate, phenylethylmethylethylcarbinyl acetate, veticol acetate, α-amylcinnamyl acetate, decahydro-β-naphthyl acetate, and furfuryl acetate.

The content of ingredient (B) (ii) is from 0.001 to 80 wit.%, preferably from 1 to 80 wt.%, more preferably from 1.5 to 60 wt.% of the fragrance composition.

As ingredient (B)(iii), i.e., the lactones, lactones having total carbon numbers of from 5 to 14, for example, compounds represented by the following formula (3): 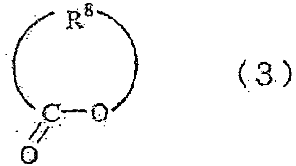 wherein R⁸ represents a hydrocarbon group having from 4 to 13 carbon atoms can be mentioned.

In formula (3), the hydrocarbon group represented by R⁸ is a linear or branched hydrocarbon group which may contain a cyclic hydrocarbon group at least as a part thereof. Specifically, linear or branched alkylene groups and alkenylene groups can be mentioned, and these alkylene groups and alkenylene groups may each contain one or more aromatic rings.

Examples of the compounds of formula (3) include γ-butyrolactone, γ-valerolactone, angelic lactone, γ-hexalactone, γ-heptalactone, γ-octalactone, γ-nonalactone, 3-methyl-4-octanolide (whisky-lactone), γ-decalactone, γ-undecalactone, γ-dodecalactone, γ-jasmolactone (7-decenolactone), δ-hexalactone, 4,6,6(4,4,6)-trimethyltetrahydropyran-2-one,δ-octalactone, δ-nonalactone, 2H-1-benzopyran-2-one, δ-decalactone, δ-2-decenolactone, δ-undecalactone, δ-dodecalactone, δ-tridecalactone, δ-tetradecalactone, lactoscatone, ε-decalactone, ε-dodecalactone, cyclohexyllactone, jasminlactone, cis-jasmonelactone, methyl-γ-decalactone, tetrahydro-6-(3-pentenyl)-2H-pyran-2-one, (E)-dec-8-en-5-olide ("JASMOLARITONE", trade name of Firmenich, Inc.), tetrahydro-6-(3-hexenyl)-2H-pyran-2-one, (Z)-undec-8-en-5-olide ("JASMOLACTONE", trade name of Bedoukian Research Inc.), menthalactone, and methyl dihydrojasmonate. Preferred are γ-octalactone, γ-nonalactone, γ-decalactone, γ-undecalactone, γ-dodecalactone, γ-jasmolactone (7-decenolactone), δ-octalactone, δ-nonalactone, 2H-1-benzopyran-2-one, δ-decalactone, δ-2-decenolactone, and δ-undecalactone.

The content of ingredient (B) (iii) is from 0.001 to 80 wt.%, preferably from 0.001 to 60 wt.%, more preferably from 0.002 to 0 40 wt.% of the fragrance composition.

As ingredient (B)(iv), i.e., the ketone compounds having a cyclic or chain skeleton and having total carbon numbers of from 5 to 14, terpenyl ketones, aliphatic linear ketones and aliphatic cyclic ketones can be mentioned.

Examples of the terpenyl ketones include camphor, carvone, dihydrocarvone, pulegone, menthone, piperitenone, diosphenol, fenchone, perpenone, geranylacetone, farnesylacetone, acetylcedrene (cedryl methyl ketone), oxocedrane (cedranone, cedrenone), acetylcaryophyllene, isolongifolanone (isolongifolaneketone), nootkatone, ionone, pseudoionone, methylionone, allylionone, irone, damascone, damascenone, isodamascone, 1-(3,3-dimethyl-6(1)-cyclohexen-1-yl)-pent-4-en-1-one ("DYNASCONE"), and trimethyl cyclohexenyl butenone ("IRITONE", trade name of International Flavors & Fragrances Inc.). Preferred are ionone, damascone, damascenone, isodamascone, and 1-(3,3-dimethyl-6(1)-cyclohexen-1-yl)-pent-4-en-1-one ("DYNASCONE").

Examples of the aliphatic linear ketones include acetoin, diacetyl, methyl amyl ketone, ethyl amyl ketone, 2-pentanone, 3-hexanone, 2-heptanone, 3-heptanone, 4-heptanone, 3-octanone, 2-nonanone, 3-nonanone, 2-undecanone, methyl isopropyl ketone, methyl hexyl ketone, methyl nonyl ketone, methylheptenone, ethyl isoamyl ketone, 2-tridecanone, mesityl oxide, butylidene acetone, methyl heptadienone, methyl heptenone, dimethyl octenone, methylene tetramethylheptanone("KOAVONE", trade name of International Flavors & Fragrances Inc.), 5-hydroxy-4-octanone (butyroin), 3-hydroxymethyl-2-nonanone, 2,3-pentanedione, 2,3-hexanedione, 3,4-hexanedione, 2,3-heptanedione, acetyl isovaleryl, 2-butyl-1,4-dioxaspiro[4.4]nonane ("JASMONE", trade name of Henkel Corporation), and 2,2,5,5-tetramethyl-4-isopropyl-1,3-dioxane.

Examples of the aliphatic cyclic ketones include amylcyclopentanone, amylcyclopentenone, 2-cyclopentylcyclopentanone, hexylcyclopentanone, butylcyclopentanone, maltol, ethyl maltol, 2,5-dimethyl-4-hydroxyfranone, 4,5-dimethyl-3-hydroxy-5H-furan-2-one ("SUGARLACTONE", trade name of Soda Aromatic Co., Ltd.), o-t-butylcyclohexanone, p-t-butylcyclohexanone, amylcyclopentanone, heptylcyclopentanone, dihydrojasmone, cis-jasmone, isojasmone, trimethylpentylcyclopentanone, 3-methyl-5-(2,3,3-trimethyl-3-cyclopentenyl)-3-penten-2-o ne ("SANDEX", trade name of Givaudan-Roure Corporation), cycloten, 3,5-dimethyl-1,2-cyclopentadione, 3,4-dimethyl-1,2-cyclopentadione, 3,3-dimethylcyclohexyl methyl ketone, 1-acetyl-3,3-dimethyl-1-cyclohexene, 2-sec-butylcyclohexanone, 3-methyl-5-propyl-2-cyclohexenone, cryptone, p-t-pentylcyclohexanone, 2,3,5-trimethyl-4-cyclohexenyl-1-methyl ketone ("METHYL CYCLOCITRON", trade name of International Flavors & Fragrances Inc.), nerone, 4-cyclohexyl-4-methyl-2-pentanone, cyclohexenyl cyclohexanone, 2,4-di-t-butylcyclohexanone ("CYCLOWOOD", trade name of Takasago International Corporation), 3-methyl-4-(2,4,6-trimethyl-3-cyclohexenyl)-3-buten-2-one ("METHYL IRITONE", trade name of International Flavors & Fragrances Inc.), allylionone, 2,6,6-trimethyl-2-cyclohexane-1,4-dione, 2-acetyl-3,3-dimethylnorbornane, 6-ethylideneoctahydro-5,8-methano-2H-1-benzopyran-2-one ("FLOREX", trade name of International Flavors & Fragrances Inc.), 4-methyltricyclo[6.2.1.02.7]undecan-5-one ("PLICATONE", trade name of Firmenich, Inc.), 6,7-dihydro-1,1,2,3,3,-pentamethyl-4(5H)-indanone ("CASHMERAN", trade name of International Flavors & Fragrances Inc.), 4(5)-acetyl-7,7,9-triemthylbicyclo[4.3.0]-1-nonene ("ATRINON", trade name of Henkel Corporation), acetylisopropylmethylbicyclooctene, 4-cyclohexyl-4-methyl-2-pentanone, p-menthen-6-ylpropanone ("NERONE", trade name of Givaudan-Roure Corporation), 2,2,5-trimethyl-5-pentylcyclopentanone, ethoxyvinyltetramethylcyclohexanone, dihydropentamethylindanone; 7-acetyl-1,2,3,4,5,6,7,8-octahydro-1,1,6,7-naphthalene ("ISO E SUPER", trade name of International Flavors & Fragrances Inc.), 2,6,7-trimethyl-1-acetyl-2,5,9-cyclododecatriene ("TRIMOFIX", trade name of International Flavors & Fragrances Inc.), acetylcedrene [ethanone, 1-(2,3,4,7,8,8a-hexahydro-3,6;8,8-tetramethyl-1H-3a,7-met hanoazulen-5-yl)-1β-methylnaphthyl ketone.

Preferred is 7-acetyl-1,2,3,4,5,6,7,8-octahydro-1,1,6,7-naphthalene ("ISO E SUPER", trade name of International Flavors & Fragrances Inc.).

The content of ingredient (B)(iv) is from 0.001 to 80 wt.%, preferably from 0.01 to 50 wt.%, more preferably from 0.1 to 30 wt.% of the fragrance composition.

As ingredient (B) (v), i.e., the aldehydes, aldehydes having total carbon numbers of from 5 to 14, for example, compounds represented by the following formula (4) :

R⁹-CHO (4)

wherein R⁹ represents a hydrocarbon group having from 4 to 13 carbon atoms and optionally containing an oxygen atom or nitrogen atom inserted in at least one carbon-carbon bond thereof can be mentioned.

The group represented by R⁹ can be a linear, branched or cyclic hydrocarbon group, or a group containing a linear, branched or cyclic hydrocarbon group with an oxygen atom or nitrogen atom inserted in at least one carbon-to-carbon bond thereof. It is to be noted that the term "hydrocarbon group" as used herein includes both saturated and unsaturated ones and the term "cyclic hydrocarbon group" as used herein includes saturated, unsaturated and aromatic, cyclic hydrocarbon groups. As the atom inserted in the at least one carbon-to-carbon bond, an oxygen atom or a nitrogen atom can be mentioned, with an oxygen atombeingpreferred. A preferred form of linkage with an oxygen atom contained therein is an ether linkage in a linear ether or cyclic ether.

Preferred examples of R⁹ include alkyl groups, alkenyl groups, cyclic hydrocarbon groups, cyclic hydrocarbyl-alkyl groups, cyclic hydrocarbyl-alkenyl groups, aromatic hydrocarbon groups, aromatic hydrocarbyl-alkyl groups, aromatic hydrocarbyl-alkenyl groups, and monoterpene and other terpene groups.

Examples of ingredient (B)(v), i.e., the aldehydes include undecenal, heptanal, octanal, undecanal, dodecanal, 2-methylundecanal, citral, geranial, neral, citronellal, 3,7-dimethyloctanal (tetrahydrocitral), hydroxycitronellal, methoxycitronellal, α-methylenecitronellal ("BENGAMAL", trade name of International Flavors & Fragrances Inc.), perilla aldehyde, methoxy dihydrocitronellal, citronellyloxy acetaldehyde, geranyloxy acetaldehyde, mirtenal, caryophyllenealdehyde, 3-ethoxy-4-hydroxybenzaldehyde, and 4-hydroxy-3-methoxybenzaldehyde. Preferred are undecenal, heptanal, octanal, undecanal, dodecanal, and 2-methylundecanal.

The content of ingredient (B)(v) is from 0.001 to 80 wt.%, preferably from 0.01 to 70 wt.%, more preferably from 0.1 to 50 wt.% of the fragrance composition.

These ingredients (B) can be used either singly or in combination. Further, the content of ingredient (B) can preferablybe from 0.001 to 80 wt.% of the fragrance composition from the viewpoint of improvements in scent and tastefulness.

Ingredient (C), i.e., the hydrocarbon having a total carbon number of from 5 to 15 can be a terpenyl hydrocarbon. Illustrative are α-pinene, β-pinene, camphene, myrcene, dihydromyrcene, limonene, dipentene, terpinene, terpinolene, carene, allo-ocimene, ocimene, α-phellandrene, p-cymene, β-caryophyllene, β-farnesene, bisabolene, cedrene, cadinene, valencene, tsujopsene, da-i-ene, and longifolene. Preferred are α-pinene, β-pinene, and limonene.

These ingredients (C) can be used either singly or in combination, although it is preferred to use two or more of them. The content of ingredient (C) in the fragrance composition differs depending upon the kinds and combination of ingredients used, but can be preferably from 0.001 to 90 wt.%, more preferably from 0.01 to 70 wt.%, even more preferably from 0.1 to 40 wt.% from the standpoint of amount sufficient to mask an acid smell, ensuring a balance with other materials, and achieving improvements in scent and tastefulness.

From the standpoint of enhancing the scent emmited, making improvements in the refreshing sensation and to give a more defined body for the fragrance, it is preferred to incorporate, in addition to the above-described ingredients, a sulfur-containing compound as an ingredient (D) in the fragrance composition according to the present invention. As the sulfur compounds, an organosulfur compound such as a thiol compound, sulfide compound, disulfide compound, thioaldehyde compound or cyclic thioether compound can be mentioned. Specific examples include propyl mercaptan, isopropyl mercaptan, 2-methyl-3-buthanethiol, allyl mercaptan, isoamyl mercaptan, thiogeraniol, limonenethiol, 8-mercaptomenthone (sulfox), phenylmercaptan, o-thiocresol, 2-ethylthiophenol, 2-naphthylmercaptan, furfuryl mercaptan, 2-methyl-3-franthiol, dimethyl sulfide, dimethyl disulfide, dimethyl trisulfide, methylpropyl disulfide, methylpropyl trisulfide, propyl disulfide, dipropyl trisulfide, diallyl trisulfide, diallyl disulfide, dibutyl sulfide, methionol, 3-methylthio-1-hexanol, methional, mentho sulfide, dithiospirolactone, furfurylmethyl sulfide, 2-methyl-5-methylthiofuran, methylfuryl disulfide, furfuryl disulfide, thiophene, tetrahydrothiophene, 3-thiophenecarboxaladehyde, 5-methyl-2-thiophenecarboxaldehyde, tetrahydrothiophen-3-one, trithioacetone, 2-methyl-4-propyl-1,3-oxathiane, thioglycolic acid, methyl ethylthioacetate, ethyl methylthioacetate, 2-methylmercaptopropionic acid, pineapple mercaptan, ethyl 3-methylthiopropionate, ethyl thioacetate, furfuryl thioacetate, furfuryl thiopropionate, methyl thiobutyrate, methylmethane thiosulfonate, allyl isothiocyanate, benzyl isothianate, thialdine, 2-methyl-4-propyl-1,3-oxathiane, p-menthane-8-thiol-3-one, p-mentene-8-thiol, and methyl β-methylthiopropionate.

From the standpoint of the amount sufficient to mask an acid smell and ensuring a balance with other materials, the content of the sulfur compound in the fragrance composition can be preferably from 0.00001 to 1 wt.%, more preferably from 0.0001 to 0.5 wt.%, even more preferably from 0.0002 to 0.4 wt.%.

The fragrance composition according to the present invention can also contain 1-(2-t-butylcyclohexyloxy)-2-butanol, dodecahydro-3a,6,6,9a-tetramethyl-naphtho[2.1-b]furan, 2-ethoxynaphthalene, 2-methoxynaphthalene, 1H-3a,7-methanoazulene, octahydro-6-methoxy-3,6,8,8-tetramethyl-, [3R-(3α,3aβ,6β,7β,8aα)], 2-oxybicyclo[2.2.2]octane, 1,3,3-trimethyl, 3,7,-dimethyl-2,6-octadienenitrile, 4,4a,5,9b-tetrahydroindeno[1,2d]-1,3-dioxine, tetrahydro-4-methyl-2-(2-methyl-1-propenyl)-2H-pyran, cyclohexanol, 3-(5,5,6-trimethylbicyclo[2.2.1]hept-2-yl)-cyclohexanol, 2-tridecenenitrile, 2-methoxy-4-allylphenol, 3-methyl-5-phenyl-1-pentanol, 1-(2-t-butylcyclohexyloxy)-2-butanol, and/or 2-methyl-4-(2,2,3-trimethyl-3-cyclopentene-1-yl)-2-buten-1-ol. From the viewpoint of imparting distinctiveness to the fragrance, the content of such an additional fragrant material may preferably be from 0.001 to 50 wt.% of the fragrance composition.

In addition, the fragrance composition according to the present invention can also contain one or more of alcohols, polyhydric alcohols and ethers.

Further, the present invention contains a fragrant material which is stable in acidic hair cosmetic compositions, especially acidic shampoos, and also contains a fragrant material which gives off a good scent from foam and also from a bottle mouth and has an excel lent lasting scent. The fragrant materials included in ingredient (E) are each characterized by the abundant release of scent from foam, the fragrant materials included in ingredient (F) are each characterized by the abundant release of a scent from a bottle mouth, and the fragrant materials included in ingredient (G) are each characterized by the goodpersistence of scent. Acombination of fragrant materials selected as desired from the ingredients (E), (F) and (G) makes it possible to obtain a well-balanced, excellent fragrance composition which is a fragrance giving off a good scent from foam and from the bottle mouth, and the persistence of scent notes.

From the viewpoint of giving off a good scent upon application of a hair cosmetic composition in which the fragrance composition according to the present invention is added, the content of ingredient (E) in the fragrance composition is from 10 wt.% to 70 wt.%, preferably from 10 wt.% to 60 wt.%, more preferably from 15 wt.% to 60 wt.%. From the viewpoint of giving off a good scent from a bottle mouth, the content of ingredient (F) in the fragrance composition is from 5 wt.% to 60 wt.%, preferably from 5 wt.% to 55 wt.%, more preferably from 10 wt.% to 50 wt.%. From the viewpoint of providing an excellent lasting scent, the content of ingredient (G) in the fragrance composition is from 10 wt.% to 70 wt.%, preferably from 10 to 60 wt.%, more preferably from 10 wt.% to 50 wt.%.

According to the present invention, a hair cosmetic composition can be formulated with the above-described fragrance composition contained therein. As the fragrance composition according to the present invention is excellent in long-term stability under high-temperature conditions and can mask a smell peculiar to an acidic hair cosmetic composition, it is useful as a fragrance composition for acidic hair cosmetic compositions, especially hair cosmetic compositions whose pHs are preferably from 1 to 5, more preferably from 2 to 4 (even more preferably, from 3 to 4). The hair cosmetic compositions of pH 1 to 5 according to the present invention can include hair cleansing compositions, rinses, treatments, conditioning agents, hair packs, hair creams, styling hair care products, hair tonics, hair restorers, hair colognes and the like, each of which has a pH of from 1 to 5, but excludes hair manicures, hair dyes, and permanent wave solutions. Among these, those used by washing them off, such as hair cleansing compositions, e.g., shampoos and conditioning shampoos, and hair rinses are preferred. It is to be noted that the term "hair cosmetic composition having a pH of 1 to 5" (25°C) as used herein means a hair cosmetic composition whose pH is from 1 to 5 when the undiluted hair cosmetic composition is diluted 20-fold with water.

A hair cosmetic composition having a pH of 1 to 5 can be prepared with a similar formula as an ordinary hair cosmetic composition except that its pH is controlled from 1 to 5. Accordingly, an oil ingredient, a conditioning agent, a humectant, a viscosity increasing agent, a viscosity controlling agent, an emulsifier, a colorant, a stabilizer, an ultraviolet absorber, a preservative, a pH adjuster and the like are added as needed in addition to one or more surfactants as a cleansing ingredient. As the surfactants, anionic surfactants, nonionic surfactants, and amphoteric surfactants can be mentioned. Examples of the anionic surfactants include polyoxyethylene alkyl ether sulfates, polyoxyethylene alkenyl ether sulfates, alkyl sulfates, and polyoxyalkylene alkyl phenyl ether sulfates, especially sulfate-type anionic surfactants such as polyoxyethylene alkyl ether sulfates and alkyl sulfates; and sulfonates and carboxylates such as alkyl sulfosuccinate salts, polyoxyalkylenealkyl sulfosuccinate salts, higher fatty acid salts, and alkane sulfonate salts.

Examples of the nonionic surfactants include polyoxyalkylene sorbitan fatty acid esters, polyoxyalkylene sorbitol fatty acid esters, polyoxyalkylene glycerol fatty acid esters, polyoxyalkylene fatty acid esters, polyoxyalkylene alkyl ethers, polyoxyalkylene alkyl phenyl ethers, polyoxyalkylene (hydrogenated) castor oils, sucrose fatty acid esters, triglycerin alkyl ethers, polyglycerin fatty acid esters, fatty acid alkanolamides, and alkyl glycosides. Among these, preferred are alkyl glycosides, polyoxyalkylene (C₈-C₂₀) fatty acid esters, polyoxyethylene sorbitan fatty acid esters, polyoxyethylene hydrogenated castor oil, and fatty acid alkanolamides. As the fatty acid alkanolamides, those having from 8 to 18 carbon atoms, especially those containing acyl groups of from 10 to 16 carbon atoms are preferred. As the fatty acid alkanolamides, either monoalkanolamides or dialkanolamides are usable, and those containing a hydroxyalkyl group of from 2 to 3 carbon atoms are preferred. Examples include oleic diethanolamide, palm kernel fatty acid diethanolamide, coconut fatty acid diethanolamide, lauric diethanolamide, polyoxyethylene coconut fatty acid monoethanolamide, coconut fatty acid monoethanolamide, lauric isopropanolamide, and lauric monoethanolamide.

As the amphoteric surfactants, betaine-type surfactants can be mentioned. Among these, more preferred are betaine alkyldimethylaminoacetates and fatty acid amidopropylbetaines, with fatty acid amidopropylbetaines being preferred. As the fatty acid amidopropylbetaines, those having 8 to 18 carbon atoms, especially those containing acyl groups of from 10 to 16 carbon atoms are preferred, with laurylamidopropyl betaine, palmkernel fatty acid amidopropyl betaine and cocamidopropyl betaine being preferred.

The surfactant can be contained preferably at from 1 to 50 wt.%, more preferably at from 8 to 30 wt.%, even more preferably at from 10 to 22 wt.% in the hair cosmetic composition.

As the cationic surfactants, mono(long-chain alkyl) quaternary ammonium salts are preferred. Specific examples include cetyltrimethylammonium chloride, stearyltrimethylammonium chloride, aralkyltrimethylammonium chloride, and behenyltrimethylammonium chloride, with behenyltrimethylammonium chloride being preferred. Further, the cationic surfactants can also include those formed by adding tertiary amines and the below-described organic salts.

As the oil ingredients, higher alcohols, lanolins, liquidparaffin, higher fatty acids, ester oils, and silicones can be mentioned. Examples of the silicones can include the following silicones:

### (1) Dimethylpolysiloxanes

Those represented by the following formula can be mentioned as examples.

(Me)₃Si-[(Me)₂SiO]_{d}-Si(Me)₃

wherein Me represents a methyl group, and d stands for a value of from 3 to 20,000.

### (2) Amino-modified silicones

Preferred examples are those having an average molecular weight of from about 3, 000 to 100, 000 and described under the name of amodimethicones in the third edition of the CTFA Cosmetic Ingredient Dictionary, U.S.A., although various amino-modified silicones are usable. Preferably, these amino-modified silicones are used as aqueous emulsions. As commercial products, "SM8704C" (product of Dow Corning Toray Silicone Co., Ltd.) and "DC929" (product of Dow Corning Corporation) can be mentioned.

### (3) Other silicones

In addition to the above-described silicones, polyether-modified silicones, methylphenylpolysiloxane, fatty-acid-modified silicones, alcohol-modified silicones, alkoxy-modified silicones, epoxy-modified silicones, fluorine-modified silicones, cyclic silicones, and alkyl-modified silicones can also be mentioned.

The oil ingredient can be contained preferably at from 0.05 to 10 wt.%, more preferably at from 0.1 to 5 wt.%, even more preferably at from 0.3 to 2 wt.% in the hair cosmetic composition.

As the conditioning agent, a cationic polymer is preferred. Examples of the cationic polymer include cationized cellulose derivatives, cationic starch, cationized guar gum derivatives, homopolymers of diallyl(quaternary ammonium) salts, diallyl(quaternary ammonium) salt/acrylamide copolymer, quaternized polyvinylpyrrolidone derivatives, polyglycol-polyamine condensation products, vinylimidazolium trichloride/vinylpyrrolidone copolymer, hydroxyethylcellulose/dimethyldiallylammonium chloride copolymer, vinylpyrrolidone/quaternized dimethylaminoethyl methacrylate copolymer, polyvinylpyrrolidone/alkyl aminoacrylate copolymers, polyvinylpyrrolidone/alkyl aminoacrylate/vinylcaprolactam copolymer, vinylpyrrolidone/methacrylamidopropyl-trimethylammonium chloride copolymer, alkylacrylamide/acrylate/alkylaminoalkyl acrylamide/polyethylene glycol methacrylate copolymers, adipic acid/dimethylaminohydroxypropyl ethylenetriamine copolymer ("CARTARETIN", product of Sandoz, Inc., U.S.A.), and cationic polymers disclosed in JP-A-53-139734 or JP-A-60-36407. Preferred examples are cationized cellulose derivatives and cationized guar gum derivatives. The conditioning agent can be contained preferably at from 0.05 to 5 wt. %, more preferably at from 0.1 to 3 wt.%, even more preferably at form 0.3 to 1 wt.% in the hair cosmetic composition.

In the hair cosmetic composition according to the present invention, an organic acid is additionally incorporated to make improvements in the finish of hair such as sleekness and style. Examples of the organic acid include carboxylic acids such as monocarboxylic acids, dicarboxylic acids, hydroxycarboxylic acids and polycarboxylic acids, and alkylphosphoric acids. Among these, carboxylic acids, especially dicarboxylic acids and hydroxycarboxylic acids are preferred. Examples of the dicarboxylic acids include malonic acid, succinic acid, glutaric acid, adipic acid, maleic acid, fumaric acid, and phthalic acid, and illustrative of the hydroxycarboxylic acids are glycolic acid, lactic acid, hydroxyacrylic acid, oxybutyric acid, glyceric acid, malic acid, tartaric acid, and citric acid. Among these, α-hydroxycarboxylic acids, especially lactic acid and malic acid are preferred.

Two or more of these organic acids can be used in combination. Further, the content of the organic acid can be preferably from 0.05 to 10 wt.%, more preferably from 0.1 to 5 wt.%, even more preferably from 0.5 to 1 wt.% of the hair cosmetic composition according to the present invention.

In the hair cosmetic composition according to the present invention, an aromatic alcohol may be additionally incorporated to make improvements in touch feel and post-shampoo sleekness. Examples of the aromatic alcohol include benzyl alcohol, benzyloxyethanol and phenoxyethanol, with benzyl alcohol and benzyloxyethanol being preferred.

Two or more of these aromatic alcohols can be used in combination. Further, the content of the aromatic alcohol can be preferably from 0.01 to 20 wt.%, more preferably from 0.1 to 10 wt.%, even more preferably from 0.5 to 5 wt.% of the hair cosmetic composition according to the present invention.

The hair cosmetic composition having a pH of 1 to 5 according to the present invention can be produced in a similar manner as conventional hair cosmetic compositions except that its pH is controlled from 1 to 5.

### Examples

In the Examples, "%" means "wt.%", and "part" or "parts" means "part by weight" or "parts by weight".

### Reference Example A Conditioning Shampoos

An unfragranced conditioning shampoo (pH 3.7) of the formula shown in Table 1 was prepared. Aliquots of the unfragranced conditioning shampoo were fragranced with 0.5 wt.% of the fragrance compositions 1 to 12 shown in Table 2 and Table 3, respectively, to formulate conditioning shampoos. Samples of the conditioning shampoos were weighed as much as 20 g each in standard 50-mL wide-mouth bottles "PS-06" (made of clear glass), and were placed in a storage cabinet controlled at 50°C. One month later, a scent given off from the surface of each shampoo was rankedby two expert panelists in accordance with the below-described ranking standard, and the average of their ranking scores was recorded. The ranking was an overall ranking on amasking effect for an acid smell, stability and the like while taking into consideration the spreading characteristics, the floating pattern and the like of the fragrance.

### Ranking standard:

- 5:: Excellent
- 4:: Good
- 3:: Satisfactory as a commercial product
- 2:: Slightly poor
- 1:: Poor

**Table 1**

| Conditioning shampoo composition (pH 3.7) | |
|---|---|
| | (wt.%) |
| Sodium POE(2) lauryl ether sulfate | 11.0 |
| Sodium lauryl sulfate | 5.0 |
| Cationized guar gum | 0.3 |
| Malic acid | 0.75 |
| Lactic acid | 0.1 |
| Sodium chloride | 0.2 |
| Benzyl alcohol | 0.5 |
| Cocoyl monoethanolamide | 1.0 |
| Dimethicone (viscosity: 100,000 cps) | 0.5 |
| Amodimethicone | 0.1 |
| Myristyl alcohol | 1.0 |
| Cetanol | 0.5 |
| Ethylene glycol distearate | 3.0 |
| Cationized hydroxyethylcellulose | 0.3 |
| Glycerol | 1.0 |
| Sodium hydroxide | q.s. to pH 3.7 |
| Deionized water | Balance |

**Table 2**

| Types | Ingredient names | Fragrance compositions | | | | | | |
|---|---|---|---|---|---|---|---|---|
| | | 1 | 2 | 3 | 4 | 5 | 6 | 7 |
| A:MUSK | Musk ketone | 0 | 3 | 3 | 3 | 3 | 3 | 3 |
| A:MUSK | "GALAXOLIDE" | 0 | 93 | 93 | 93 | 93 | 93 | 93 |
| B-i:ESTER | Cis-3-hexenyl salicylate | 0 | 0 | 15 | 0 | 0 | 15 | 15 |
| B-i:ESTER | Ethyl 2-methylbutyrate (DPG 10%) | 0 | 0 | 5 | 0 | 0 | 5 | 5 |
| B-i:ESTER | "FRUITATE" (ethyl tricyclodecanylcarboxylate) | 0 | | | 0 | 0 | 5 | 5 |
| B-i:ESTER | Tricyclodecenyl propionate | 0 | 0 | 20 | 0 | 0 | 20 | 20 |
| B-i:ESTER | Methyl salicylate (DPG 10%) | 0 | | | 0 | 0 | 5 | 5 |
| B-ii:ESTER | o-t-Butyl cyclohexylacetate | 0 | 0 | 1.5 | 0 | 0 | 15 | 15 |
| B-iii:KETONE | "ADMASCENONE" | 0 | 0 | | 0.5 | 0 | 0 | 0.5 |
| B-iii:KETONE | "α-DAMASCONE" | 0 | 0 | 0 | 1.5 | 0 | 0 | 1.5 |
| B-iii:KETONE | β-Ionone | 0 | 0 | 0 | 20 | 0 | 0 | 20 |
| B-iii:KETONE | Methylionone-G | 0 | 0 | 0 | 25 | 0 | 0 | 25 |
| C:TERPENE | Limonene | 0 | 0 | 0 | 0 | 25 | 25 | 25 |
| SOLVENT | Dipropylene glycol | 350 | 244 | 191 | 207 | 229 | 164 | 117 |
| | FORMULA*^{note 1} | 650 | 650 | 650 | 650 | 650 | 650 | 650 |
| | Total (parts by weight) | 1000 | 1000 | 1000 | 1000 | 1000 | 1000 | 1000 |

| | | | | | | | | |
|---|---|---|---|---|---|---|---|---|
| FORMULA*^{note 1} Fragrance formula formed primarily of alcohols and containing aldehydes, dipropylene glycol, etc. | | | | | | | | |

**Table 3**

| Types | Ingredient names | Fragrance compositions | | | | |
|---|---|---|---|---|---|---|
| | | 8 | 9 | 10 | 11 | 12 |
| - | Hexyl acetate | 5 | 0 | 0 | 0 | 0 |
| - | Iso-amyl acetate | 2 | 0 | 0 | 0 | 0 |
| - | Indole (DPG 10%) | 5 | 0 | 0 | 0 | 0 |
| A | Musk ketone | 3 | 0 | 3 | 3 | 3 |
| A | "GALAXOLIDE" | 185 | 0 | 185 | 185 | 185 |
| B-i | "FRUITATE" | 0 | 0 | 0 | 5 | 5 |
| B-i | Tricyclodecenyl propionate | 0 | 0 | 0 | 20 | 20 |
| B-i | Methyl salicylate (DPG 10%) | 0 | 0 | 0 | 5 | 5 |
| B-i | Cis-3-hexenyl salicylate | 0 | 0 | 0 | 15 | 15 |
| B-i | Ethyl 2-methylbutyrate (DPG 10%) | 0 | 0 | 0 | 0 | 5 |
| B-ii | o-t-Butyl cyclohexylacetate | 0 | 0 | 0 | 15 | 15 |
| B-iii | G-Decanolactone | 0 | 20 | 0 | 20 | 20 |
| B-iv | "DAMASCENONE" | 0.5 | 0 | 0 | 0.5 | 0.5 |
| B-iv | "d-DAMASCONE" | 1.5 | 0 | 0 | 1.5 | 1.5 |
| B-iv | β-Ionon | 20 | 0 | 0 | 20 | 20 |
| B-iv | Methylionon-G | 25 | 0 | 0 | 25 | 25 |
| B-v | α-Methyl-1,3-benzodioxol-5-propanal | 0 | 0 | 10 | 10 | 10 |
| B-v | Decanal (DPG 10%) | 0 | 0 | 2 | 2 | 2 |
| B-v | Dodecanal (DPG 10%) | 0 | 0 | 1 | 0 | 1 |
| B-v | Hexylcinnamic aldehyde | 0 | 0 | 90 | 90 | 90 |
| B-v | α-Methyl-4-(1-methylethyl)-benzenepropanal | 0 | 0 | 15 | 15 | 15 |
| B-v | p-t-Butyl-α-methylhydrocinnamic aldehyde | 0 | 0 | 140 | 140 | 140 |
| C | Limonene | 25 | 0 | 0 | 25 | 25 |
| D | p-Menthane-8-thiol-3-one (1% DPG) | 0 | 0 | 0 | 0 | 1 |
| Ether | Dodecahydro-3a,6,6,9a-tetramethyl-naphtho[2.1-b]furan | 0 | 0 | 0 | 0 | 0.3 |
| | FORMULA*^{note 2} | 340 | 340 | 340 | 340 | 340 |
| Solvent | Dipropylene glycol | 388 | 640 | 402 | 57 | 55.7 |
| | Total (parts by weight) | 1000 | 1000 | 1000 | 1000 | 1000 |

| | | | | | | |
|---|---|---|---|---|---|---|
| FORMULA*^{note 2} Fragrance formula formed primarily of alcohols and containing solvents such as dipropylene glycol, etc. | | | | | | |

The results are shown in Table 4.

**Table 4**

| Results of Ranking in Stability and Masking Effect | | | | | | | | | | | | | |
|---|---|---|---|---|---|---|---|---|---|---|---|---|---|
| | | Comp. Exs. | | Reference Examples | | | | | Comp. Exs. | | Reference Examples | | |
| | | 1 | 2 | 1 | 2 | 3 | 4 | 5 | 3 | 4 | 6 | 7 | 8 |
| Composition of Table 1 | | 99.50% | 99.50% | 99.50% | 99.50% | 99.50% | 99.50% | 99.50% | 99.50% | 99.50% | 99.50% | 99.50% | 99.50% |
| Fragrance composition 1 | | 0.50% | | | | | | | | | | | |
| Fragrance composition 2 | | | 0.50% | | | | | | | | | | |
| Fragrance composition 3 | | | | 0.50% | | | | | | | | | |
| Fragrance composition 4 | | | | | 0.50% | | | | | | | | |
| Fragrance composition 5 | | | | | | 0.50% | | | | | | | |
| Fragrance composition 6 | | | | | | | 0.50% | | | | | | |
| Fragrance composition 7 | | | | | | | | 0.50% | | | | | |
| Fragrance composition 8 | | | | | | | | | 0.50% | | | | |
| Fragrance composition 9 | | | | | | | | | | 0.50% | | | |
| Fragrance composition 10 | | | | | | | | | | | 0.50% | | |
| Fragrance composition 11 | | | | | | | | | | | | 0.50% | |
| Fragrance composition 12 | | | | | | | | | | | | | 0.50% |
| | | | | | | | | | | | | | |
| changes with time | Room temρ.1day | 1 | 3 | 3.5 | 4 | 3.5 | 4 | 5 | 4 | 2 | 4 | 5 | 5 |
| | 50°C 1 month later | <1 | 2 | 3 | 4 | 3.5 | 3.5 | 4.5 | 1 | 2 | 3.5 | 4.5 | 4.5< |
| Performance (masking performance) | | 1 | 3 | 3.5 | 4 | 3.5 | 4 | 5 | 4 | 2 | 4 | 5 | 5 |

As shown in FIG. 4, the conditioning shampoos formulated by fragrancing the aliquots of the unfragranced conditioning shampoo of Table 1 with the fragrance compositions of reference Examples 1 to 8 according to the present invention were acknowledged to have clear advantages in changes with time and performance (masking performance) over the conditioning shampoos formulated by fragrancing the aliquots of the unfragranced conditioning shampoo of Table 1 with the fragrance compositions of Comparative Examples 1 to 4 from a comparison therebetween.

In the case of reference Example 2, for example, the change with time upon elapsed time of 1 month at 50°C were ranked "4" (good) like the change with time one day after the formulation owing to the inclusion of the fragrance composition 4 according to the present invention, thereby demonstrating excellent stability at high temperatures. In the case of reference Example 5, the change with time upon elapsed time of 1 month at 50°C were ranked "4.5" as compared with "5" one day after the formulation owing to the inclusion of the fragrance composition 7 according to the present invention, thereby also demonstrating excellent stability at high temperatures.

### Reference Example B Clear Shampoo

| | (wt.%) |
|---|---|
| Sodium POE(2) lauryl ether sulfate | 10.0 |
| Myristyl alcohol | 0.5 |
| Cationized hydroxyethylcellulose | 0.2 |
| Laurylamidopropyl betaine | 0.5 |
| Cocoyl monoethanolamide | 0.3 |
| Malic acid | 0.75 |
| Glycerol | 1.0 |
| Deionized water | Balance |

A clear shampoo formulated by adding the fragrance composition 7 to the above-described composition (pH 3.7) such that its content became 0.5 wt.% was free of any acid smell, and retained the fragrance over a long time.

### Reference Example C Anti-dandruff Shampoo

| | (wt.%) |
|---|---|
| Sodium POE(2) lauryl ether sulfate | 10.0 |
| Sodium lauryl sulfate | 5.5 |
| Myristyl alcohol | 1.0 |
| Cetanol | 0.5 |
| Cationized hydroxyethylcellulose | 0.3 |
| Cationized guar gum | 0.3 |
| Cocoyl monoethanolamide | 0.5 |
| Dimethicone (polymerization degree:2,000) | 0.5 |
| Dimethicone (polymerization degree:200) | 0.5 |
| Malic acid | 0.7 |
| Benzyloxyethanol | 0.5 |
| Ethylene glycol distearate | 3.0 |
| Cocoyl benzalconium chloride | 0.5 |
| Glycerol | 1.0 |
| Sodium chloride | 0.2 |
| Deionized water | Balance |

Ashampoo formulated by adding the fragrance composition 7 to the above-described composition (pH 3.7) such that its content became 0. 5 wt. % was free of any acid smell, and retained the fragrance over a long time.

### Reference Example D Conditioner

| | (wt.%) |
|---|---|
| Lactic acid | 4 |
| Polypropylene glycol (m.w. 400) | 1 |
| Behenyltrimethylammonium chloride | 1.7 |
| Stearyltrimethylammonium chloride | |
| Behenyl alcohol | 5.1 |
| Methylpolysiloxane ("SH500-5000CS") | 3 |
| Isopropyl palmitate | 1 |
| Dipentaerythritol fatty acid ester | 0.1 |
| Benzyloxyethanol | 0.3 |
| Hydroxyethylcellulose | 0.2 |
| Polyethylene glycol (m.w. 100,000) | |
| 48% Sodium hydroxide | 0.2 |
| Deionized water | Balance |

A conditioner formulated by adding the fragrance composition 7 to the above-described composition (pH 3.3) such that its content became 0.5 wt.% was free of any acid smell, . and retained the fragrance over a long time.

### Example. E

With respect to each fragrant material, ranking was conducted in accordance with the ranking methods and ranking standards to be described hereinafter.

### <Ranking methods & ranking standards>

### a. Scent from foam

### (1) Ranking method

1. Each fragrant material (0.5 g) was added to a shampoo base (99.5 g) to fragrance the shampoo base (0.5 wt. %).
2. On the following day, 0.5 g of the thus-fragranced shampoo base was weighed in a 100-cc container, and water was then added to produce a total weight of 10 g so that a 5% aqueous solution was obtained.
3. The aqueous solution was lathered for 1 minute with a commercial electric beater.
4. A scent given off from the foam was ranked in accordance with the following standard.

### (1) Ranking standard

5: A scent is given off very well so that the smell of the cosmetic base (acid smell) is completely masked.
4: A scent is given off well so that the smell of the cosmetic base (acid smell) is masked well.
3: A scent is given off well, but the masking of the smell of the cosmetic base (acid smell) is a little insufficient.
2: A scent is given off, but the smell of the cosmetic base (acid smell) is also recognized.
1: A scent is poorly given off, and the smell of the cosmetic base (acid smell) is recognized.

### b. Scent from the bottle mouth

### (1) Ranking method

1. Each fragrant material (0.5 g) was added to a shampoo base (99.5 g) to fragrance the shampoo base (0.5 wt.%).
2. The thus-formulated shampoo was poured in 20 g aliquots into glass bottles of 50 g capacity (standardized wide-mouth bottles "PS-06", made of clear glass), followed by capping the glass bottles.
3. On the following day, each bottle was uncapped to rank a scent from the bottle mouth.

### (2) Ranking standard

5: A scent is given off very well so that the smell of the cosmetic base (acid smell) is completely masked.
4: A scent is given off well so that the smell of the cosmetic base (acid smell) is masked well.
3: A scent is given off well, but the masking of the smell of the cosmetic base (acid smell) is a little insufficient.
2: A scent is given off, but the smell of the cosmetic base (acid smell) is also recognized.
1: A scent is poorly given off, and the smell of the cosmetic base (acid smell) is recognized.

### c. Persistence of scent

### (1) Ranking method

1. Each fragrant material (0. 5 g) was added to a shampoo base (99.5 g) to fragrance the shampoo base (0.5 wt.%).
2. On the following day, 2.5 g of the thus-formulated, fragranced sample were applied to a tress of false hair.
3. The tress of false hair was washed for 1 minute and rinsed (with running water) for 1 minute, and was then lightly dried with a towel.
4. The tress of false hair was allowed to dry naturally, and one day later, the scent was ranked.

### (2) Ranking standard

5: A scent remains very well so that the smell of the cosmetic base is masked sufficiently.
4 : A scent remains well so that the smell of the cosmetic base is masked sufficiently.
3: A scent remains, but the masking of the smell of the cosmetic base is a little insufficient.
2: A scent remains only faintly so that the masking of the smell of the cosmetic base is insufficient.
1: No scent remains at all.

### d. Changes in odor with time (stability)

### (1) Ranking method

Each fragrant material was added to an acidic shampoo base. Samples were stored for 1 month in constant-temperature chambers controlled at 0°C and 50°C, respectively. After the temperatures of the respective samples were allowed to return to room temperature one month later, a scent from the sample stored at 50°C was ranked in comparison with a scent from the sample stored at 0°C in accordance with the below-described standard. The scent emitted from the sample stored at 0°C was taken as standard. The ranking was performed by a panel of 3 to 6 experts, and the average of their scores was indicated with 0.5 increments.

### (2) Ranking standard

5: Substantially the same in both odor quality and intensity compared with the standard (the sample stored at 0°C).
4: Slightly changed in both odor quality and intensity compared with the standard, but still good as a scent of merchandise.
3: Changed in both odor quality and intensity compared with the standard, but still acceptable practically as a scent of merchandise.
2: Obviously changed in both odor quality and intensity compared with the standard.
1: Pronouncedly changed in both odor quality and intensity compared with the standard.

### <Ranking results>

Tables 5 to 13 show fragrant materials which were found to have good stability and also to be good in any of the properties of scent from foam, scent from a bottle mouth and the persistence of a scent, based on the results of the above-described rarikings.

**Table 5**

| Names of fragrant materials | Stability | Scent from bottle mouth | Scent from foam | Persistence of scent | Ingredient |
|---|---|---|---|---|---|
| α-Amylcinnamic aldehyde | 4.5 | 4.0 | 4.0 | 3.0 | E |
| Acetophenone | 4.5 | 5.0 | 4.0 | 4.0 | E |
| Decanal | 5.0 | 4.0 | 5.0 | 2.5 | E |
| Undecanal | 5.0 | 4.0 | 4.5 | 2.5 | E |
| Undecenal | 5.0 | 4.5 | 5.0 | 2.5 | E |
| Dodecanal | 5.0 | 4.0 | 5.0 | 2.5 | E |
| 2-Methylundecanal | 4.5 | 4.0 | 4.5 | 2.5 | E |
| Octanal | 5.0 | 4.5 | 4.5 | 2.5 | E |
| Nonanal | 5.0 | 4.5 | 4.5 | 2.5 | E |
| Allylcyclohexyl propionate | 0.0 | 0.0 | 0.0 | 0.0 | E |
| Allyl phenoxyacetate | 0.0 | 0.0 | 0.0 | 0.0 | E |
| Allylphenoxyacetatic acid | 3.5 | 4.5 | 4.0 | 4.0 | |
| Anisaldehyde | 3.5 | 4.0 | 4.0 | 2.5 | E |
| Anisyl acetate | 3.5 | 3.5 | 3.5 | 3.5 | E |
| Anisyl acetone | 4.0 | 3.5 | 4.0 | 4.0 | E |
| Borneol | 4.0 | 4.5 | 4.5 | 2.0 | E |
| 3-(p-Tert-butylphenyl)-propanal ("BOURGENAL", trade name of Quest) | 4.0 | 4.0 | 4.5 | 2.5 | E |
| 7-Methyl-3,5-dihydro-2H-benzodioxepin-3-one ("CALONE", trade name of Pfizer) | 3.0 | 5.0 | 5.0 | 3.5 | E |
| Cinnamyl acetate | 3.5 | 4.0 | 4.0 | 4.0 | E |
| Cis-4-decenal | 4.5 | 5.0 | 5.0 | 2.5 | E |
| Cis-jasmone | 3.5 | 3.0 | 3.5 | 3.0 | E |

**Table 6**

| Names of fragrant materials | Stability | Scent from bottle mouth | Scent from foam | Persistence of scent | Ingredient |
|---|---|---|---|---|---|
| citronellyloxy acetaldehyde | 4.0 | 4.0 | 4.5 | 2.5 | E |
| Dodecanenitrile ("CLONAL", trade name of IFF) | 4.0 | 4.5 | 4.5 | 3.5 | E |
| 2-Methyl-3-(isopropylphenyl)-propanal (cyclamen aldehyde) | 3.5 | 3.5 | 4.0 | 2.0 | E |
| Dihydrojasmone | 3.5 | 4.0 | 4.5 | 3.0 | E |
| Methyl dihydrojasmonate | 4.5 | 3.5 | 3.5 | 2.0 | E |
| Dihydromircenol | 3.0 | 4.0 | 4.0 | 2.0 | E |
| Tricyclodecylidene-4-butanal ("DUPICAL", trade name of Quest) | 4.0 | 4.5 | 5.0 | 2.0 | E |
| Ethyl 2-cyclohexylpropionate ("ETHYL POIRENATE", trade name of Kao) | 4.0 | 4.5 | 4.0 | 4.0 | E |
| Ethyl 2,6,6-trimethyl-1,3-cyclohexadiene-1-carboxylate ("ETHYL SUFRANATE", trade name of Quest) | 4.0 | 4.5 | 4.5 | 4.0 | E |
| Ethyl isovalerate | 3.5 | 4.0 | 4.0 | 1.0 | E |
| Ethyl linalool | 3.0 | 2.5 | 3.0 | 2.0 | E |
| Ethyl vanillin | 3.5 | 4.5 | 5.0 | 2.0 | E |
| Eugenol | 4.5 | 4.0 | 4.0 | 3.5 | E |
| 6-Ethylideneoctahydro-5,8-methano-2H-1-benzopyran-2-one ("FLOREX", trade name of Firmenich) | 4.0 | 4.0 | 4.5 | 2.5 | E |
| Ethyl tricyclodecan-2-yl-carboxylate ("FRUITATE", trade name of Kao) | 5.0 | 3.5 | 4.0 | 3.5 | E |
| Franeol | 4.0 | 5.0 | 5.0 | 1.5 | E |
| Ethyl-3-(bicyclo[2.2.1]hepto-5-en-2-yl)-3-methyl glycidate ("GLYCOMEL", trade name of Firmenich) | 3.0 | 3.5 | 4.0 | 4.0 | |
| 2-Methyl-3-(3,4-methylenedioxyphenyl)-propanol ("HELIONAL", trade name of IFF) | 3.5 | 4.0 | 4.5 | 3.0 | E |
| cis-3-hexenyl methylcarbonate ("LIFFAROME", trade name of IFF) | 2.0 | 3.0 | 4.0 | 2.5 | E |
| 2-Methyl-3-(4-tert-butylphenyl)-propanal ("LILIAL", Givaudan) | 3.5 | 3.5 | 4.0 | 2.0 | E |
| Linalool | 3.5 | 4.0 | 4.0 | 3.5 | |
| 4(3)-(4-Hydroxy-4-methylpentyl)-3-cyclohexene-1-carboxaldehyde ("LYRAL", trade name of IFF) | 2.5 | 2.5 | 4.0 | 2.5 | E |
| Methyl anthranilate | 4.0 | 4.5 | 4.5 | 4.0 | E |
| Methyl β-naphthyl ketone | 4.5 | 4.0 | 4.5 | 4.5 | E |
| 4(3)-(4-Methyl-3-pentenyl)-3-cyclohexene-1-carboxaldehyde ("MYRAC ALDEHYDE", trade name of IFF) | 4.0 | 4.5 | 5.0 | 2.5 | E |

**Table 7**

| Names of fragrant materials | Stability | Scent from bottle mouth | Scent from foam | Persistence of scent | Ingredient |
|---|---|---|---|---|---|
| 2-(2-(4-Methyl-3-cyclohexen-1-yl)propyl)cyclopentanone ("NECTARYL", trade mark of Givaudan) | 4.5 | 4.0 | 4.5 | 4.0 | E |
| Nookkatone | 3.5 | 3.5 | 3.5 | 3.0 | E |
| Phenylacetaldehyde | 1.0 | 4.5 | 5.0 | 2.5 | E |
| 2-Cyclohexylpropanal ("POLLENAL II", trade name of Kao) | 4.5 | 4.0 | 4.0 | 2.0 | E |
| 8-Methoxytricyclodecane-4-carboxaldehyde ("SCENTENAL", trade name of Firmenich) | 4.0 | 4.0 | 5.0 | 3.0 | E |
| Thymol | 4.5 | 4.5 | 4.5 | 2.0 | E |
| Trans-2-decenal | 4.5 | 5.0 | 5.0 | 2.5 | E |
| Trans-2-hexanal | 4.5 | 4.5 | 5.0 | 2.5 | E |
| Tricyclodecenyl acetate | 4.0 | 3.0 | 4.0 | 4.0 | E |
| Tricyclodecenyl propionate | 4.0 | 3.5 | 4.0 | 4.0 | E |
| 1-Acetyl-2,6,10-trimethylcyclododeca-2,5,9-triene ("TRIMOFIX", trade name of IIF) | 4.0 | 2.5 | 3.0 | 3.0 | E |
| Vanillin | 3.5 | 4.5 | 4.5 | 2.5 | E |
| 1-Octen-3-yl acetate | 3.5 | 4.0 | 3.0 | 2.0 | F |
| 2,5-Decadienal | 3.0 | 4.5 | 4.5 | 2.5 | F |
| 2,4-Octadienal | 3.0 | 5.0 | 4.0 | 2.5 | F |
| 2,6-Nonadienal | 3.0 | 5.0 | 5.0 | 2.5 | F |
| 1,3-Oxathiane 2-methyl-4-propionate | 3.0 | 4.0 | 2.5 | 2.0 | F |
| 2-Methylbutyl 2-methylbutyrate | 3.0 | 4.0 | 1.0 | 2.0 | F |
| 2-Methylbutyl isovalerate | 3.5 | 4.0 | 1.5 | 2.5 | F |
| 2-Methylbutyl valerate | 3.0 | 3.5 | 1.5 | 2.0 | F |
| Acetophenone | 4.0 | 5.0 | 3.5 | 3.5 | F |
| Acetylcedrene [ethanone, 1-(2,3,4,7,8,8a-hexahydro-3,6,8,8-tetramethyl-1H-3a, 7-methan oazulen-5-yl)-]β-methylnaphthyl ketone | 4.0 | 3.5 | 3.0 | 4.5 | F |
| α-Damascon | 4.0 | 4.0 | 4.5 | 3.0 | F |
| Hexanol | 4.5 | 4.0 | 1.5 | 2.5 | F |
| Hexanal | 5.0 | 5.0 | 4.0 | 2.5 | F |

**Table 8**

| Names of fragrant materials | Stability | Scent from bottle mouth | scent from foam | Persistence of scent | Ingredient |
|---|---|---|---|---|---|
| Heptanal | 5.0 | 4.5 | 4.0 | 2.5 | F |
| Allylionone | 3.5 | 4.5 | 3.5 | 3.0 | F |
| Allyl heptanoate | 3.0 | 3.5 | 3.0 | 1.5 | F |
| Allyl hexanoate | 3.0 | 4.5 | 1.5 | 1.5 | F |
| α-Pinene | 3.0 | 3.0 | 2.5 | 1.0 | F |
| α-Terpinene | 3.0 | 4.5 | 3.0 | 1.0 | F |
| β-Damascon | 4.0 | 3.0 | 3.5 | 3.0 | F |
| Benzaldehyde | 4.5 | 4.5 | 2.0 | 2.0 | F |
| Benzyl acetate | 3.0 | 4.5 | 4.0 | 3.0 | F |
| Benzyl propionate | 3.0 | 4.0 | 3.5 | 3.0 | F |
| Camphor | 4.0 | 4.5 | 2.0 | 2.0 | F |
| 2-Methyl-3-(para-methoxyphenylt-propylaldehyde ("CANTHOXAL", trade name of IFF) | 3.0 | 3.5 | 2.5 | 2.0 | F |
| Cedrene | 4.0 | 4.5 | 2.0 | 4.0 | F |
| 3-Proylbicyclo(2.2.1)hept-5-ene-2-carbaldehyde ("CHRYSANTHAL", trade name of Quest) | 4.0 | 5.0 | 4.5 | 3.5 | F |
| Cinnamic alcohol | 4.5 | 4.0 | 3.5 | 2.5 | F |
| Cis-3-hexenol | 4.5 | 4.5 | 4.5 | 3.0 | F |
| Cis-3-hexenyl propionate | 3.0 | 5.0 | 3.0 | 2.0 | F |
| cis-jasmone | 3.5 | 3.5 | 3.5 | 3.0 | F |
| Citral | 4.5 | 4.5 | 4.0 | 2.5 | F |
| Citronellal | 4.0 | 4.5 | 3.0 | 2.5 | F |
| Citronellol | 3.0 | 3.5 | 3.5 | 2.5 | F |
| Citronellyl nitrile | 4.5 | 4.5 | 3.0 | 4.0 | F |
| Thiol addition product of limonene (for example, "CORPS PAMPLEMOUSSE", trade name of Prodasynth) | 4.0 | 4.0 | 2.5 | 2.0 | F |
| Methyl cyclopentylidenacetate | 3.5 | 4.5 | 1.0 | 2.0 | F |
| Dimethlbenzylcarbinyl acetate | 4.0 | 3.5 | 3.5 | 3.5 | F |

**Table 9**

| Names of fragrant materials | Stability | Scent from bottle mouth | Scent from foam | Persistence of scent | Ingredient |
|---|---|---|---|---|---|
| 1-(2,6,6-Trimethyl-1,3-cyclohexadien-1-yl)-2-buten-1-one ("DAMASCENONE", trade name of Firmenich) | 4.0 | 4.5 | 4.0 | 3.0 | F |
| 1-(2,6,6-Trimethyl-3-cyclohexen-1-yl)-2-buten-1-one ("DELTA-DAMASCONE", trade name of IFF) | 4.0 | 4.5 | 4.5 | 3.0 | F |
| Dihydrojasmone | 3.5 | 4.5 | 4.0 | 3.5 | F |
| Ethyl maltol | 4.0 | 5.0 | 4.5 | 1.5 | F |
| Ethyl trimethylhexanoate | 4.0 | 3.0 | 0.5 | 1.5 | F |
| Ethyl 2-methylbutylate | 3.0 | 4.0 | 1.0 | 1.0 | F |
| Ethyl butyrate | 3.0 | 4.5 | 3.5 | 1.0 | F |
| Ethyl heptanoate | 4.0 | 3.5 | 1.0 | 1.0 | F |
| Ethyl hexanoate | 4.0 | 4.5 | 1.5 | 1.0 | F |
| Ethyl nonanoate | 4.0 | 3.5 | 1.5 | 1.0 | F |
| Ethyl octanoate | 4.0 | 3.0 | 1.5 | 1.0 | F |
| Ethyl propionate | 2.5 | 5.0 | 2.0 | 1.0 | F |
| Ethyl valerate | 3.0 | 4.5 | 3.5 | 1.0 | F |
| Fenchone | 4.0 | 5.0 | 2.5 | 3.0 | F |
| p-Ethyl-2,2-dimethylhydrocinnamaldehyde ("FLORALOZONE", trade name of IFF) | 3.0 | 4.5 | 4.0 | 2.5 | F |
| Ethyl 2-tert-butylcyclohexylcarbonate ("FLORAMAT", trade name of Kao) | 4.0 | 4.0 | 2.5 | 3.5 | E |
| 2-sec-Butylcyclohexanone ("FRESCOMENTHE", trade name of Givaudan) | 4.0 | 3.5 | 2.5 | 2.5 | F |
| Ethyl-2-methyl-1,3-dioxolan-2-acetate | 3.5 | 3.0 | 1.0 | 3.0 | F |
| Geraniol | 4.0 | 3.5 | 3.5 | 2.5 | F |
| Geranyl nitrile | 4.5 | 5.0 | 4.0 | 4.5 | F |
| 3-Methyl-5-prop-2-cyclohexen-1-one ("GRAVENONE", trade name of Dragoco) | 3.5 | 4.0 | 2.5 | 2.0 | F |
| Hexylcinnamic aldehyde | 3.0 | 3.5 | 3.5 | 3.0 | F |
| Heptyl cyclopentanone | 4.0 | 4.0 | 4.5 | 4.0 | F |
| Iron | 4.5 | 4.5 | 3.0 | 2.0 | F |
| Isobutyl salicylate | 4.0 | 3.5 | 2.5 | 3.5 | F |

**Table 10**

| Names of fragrant materials | Stability | Scent from bottle mouth | Scent from foam | Persistence of scent | Ingredient |
|---|---|---|---|---|---|
| 1-(2,4,4-Trimethyl-2-cyclohexen-1-yl)-2-buten-1-one ("ISO-DAMASCONE", trade name of Dragoco) | 4.0 | 4.0 | 3.0 | 3.0 | F |
| L-Carvone | 4.0 | 5.0 | 4.5 | 2.0 | F |
| 3,7-Dimethyl-2(3),6-nonadienenitrile ("LEMONILE", tradename of Givaudan) | 4.0 | 4.5 | 3.5 | 4.5 | F |
| Limonene | 4.5 | 3.5 | 2.0 | 1.0 | F |
| L-Menthol | 4.0 | 3.0 | 2.5 | 2.0 | F |
| Menthone | 4.0 | 5.0 | 3.5 | 2.0 | F |
| Maltol | 4.0 | 4.5 | 4.0 | 1.0 | F |
| 3-Methyl-5-phenylpentanal ("MEFRANAL", trade name of Quest) | 4.0 | 4.0 | 3.5 | 2.5 | F |
| Methyl benzoate | 3.5 | 4.5 | 2.0 | 3.0 | F |
| Methyl butyrate | 3.0 | 4.5 | 1.0 | 1.0 | F |
| Methyl dihydrojasmonate | 3.5 | 4.0 | 2.0 | 4.0 | F |
| Methyl geranylate | 4.0 | 3.0 | 1.5 | 2.0 | F |
| Methyl octanoate | 4.0 | 4.0 | 1.5 | 2.0 | F |
| Methyl salicylate | 4.0 | 4.5 | 3.5 | 2.0 | F |
| Methyl valerate | 4.0 | 5.0 | 3.0 | 1.0 | F |
| Butyl butyrate | 4.0 | 3.5 | 1.0 | 1.0 | F |
| Hexyl acetate | 3.5 | 4.0 | 1.5 | 1.0 | F |
| Hexylcyclopentanone | 4.0 | 3.5 | 3.5 | 3.0 | F |
| Amyl valerate | 4.0 | 3.5 | 1.5 | 1.5 | F |
| Amyl butyrate | 3.5 | 4.0 | 1.0 | 1.0 | F |
| Amyl propionate | 3.0 | 4.5 | 2.0 | 1.0 | F |
| Orthomethoxycinnamic aldehyde | 2.5 | 4.0 | 4.0 | 2.0 | F |
| o-Tert-butylcyclohexyl acetate | 4.0 | 3.5 | 2.0 | 3.0 | F |
| Para-Amylcyclohexanone | 4.0 | 3.5 | 2.5 | 3.0 | F |
| Paracymene | 4.0 | 4.5 | 1.5 | 1.0 | F |

**Table 11**

| Names of fragrant materials | Stability | Scent from bottle mouth | Scent from foam | Persistence of scent | Ingredient |
|---|---|---|---|---|---|
| Phenylethyl alcohol | 4.0 | 3.5 | 3.0 | 2.5 | F |
| Phenylethyl propionate | 3.0 | 4.0 | 3.5 | 2.5 | F |
| Phenylhexanol | 4.5 | 2.5 | 2.0 | 2.5 | F |
| Phenylpropyl acetate | 3.0 | 3.5 | 3.0 | 3.0 | F |
| 4-Methyltricyclo[6.2.1.02.7]undecan-5-one ("PLICATONE", trade name of Firmenich) | 3.0 | 3.0 | 2.5 | 2.5 | F |
| p-Menthane-8-thiol-3-one | 4.0 | 5.0 | 4.5 | 2.5 | F |
| p-Tert-butylcyclohexyl acetate | 4.0 | 3.5 | 2.0 | 3.5 | F |
| Styrallyl acetate | 3.5 | 5.0 | 3.0 | 3.0 | F |
| Terpineol | 4.0 | 3.5 | 2.0 | 2.0 | F |
| Terpinolene | 3.0 | 3.5 | 1.5 | 1.5 | F |
| Terpinyl propionate | 3.0 | 3.5 | 2.0 | 2.5 | F |
| Trans-2-hexanal | 3.0 | 4.5 | 3.0 | 2.5 | F |
| Trans-2-hexenyl acetate | 3.5 | 3.5 | 1.5 | 1.5 | F |
| Trimethylhexanal | 4.5 | 4.5 | 3.5 | 2.5 | F |
| 2,4-Dimethyl-3-cyclohexenylcarboxaldehyde ("TRIPLAL", trade name of IFF) | 4.5 | 5.0 | 4.5 | 2.5 | F |
| 2,2,5-Trimethyl-5-pentylcyclopentanone ("VELOUTONE", trade name of Firmenich) | 4.0 | 3.5 | 2.0 | 2.5 | F |
| 4-Cyclohexyl-4-methyl-2-pentanone ("VETIVERTONE", trade name of Quest) | 3.5 | 4.0 | 2.5 | 3.0 | F |
| Vetiveryl acetate | 3.5 | 4.0 | 3.5 | 3.5 | F |
| Amylcyclopentane | 4.0 | 4.5 | 4.0 | 3.0 | F |
| Allyl anthranillate, | 3.5 | 2.5 | 2.5 | 4.0 | G |
| 1-(2-Tert-butylcyclohexyloxy)-2-butanol ("AMBER CORE", trade name of Kao) | 4.0 | 3.5 | 3.0 | 4.0 | G |
| 7-cyclohexadecenolide (ambrettolide) | 2.5 | 2.5 | 3.5 | 4.0 | G |
| (3α,6,6,9α-tetramethyldodecahydronaphtho[2.1-b]furan ("AMBROXANE", trade name of Kao) | 4.5 | 3.5 | 3.5 | 4.0 | G |

**Table 12**

| Names of fragrant materials | Stability | Scent from bottle mouth | Scent from foam | Persistence of scent | Ingredient |
|---|---|---|---|---|---|
| 2-Ethyl-4-(2,2,3-trimethyl-3-cyclopenten-1-yl)-2-buten-1-ol ("BACDANOL", trade name of IFF; "BANGALOL", trade name of Givaudan) | 4.0 | 2.5 | 3.5 | 4.5 | G |
| Benzophenone | 5.0 | 3.0 | 3.5 | 4.0 | G |
| Benzyl alcohol | 4.5 | 4.0 | 3.5 | 4.0 | G |
| Caryophyllene | 4.0 | 1.5 | 1.0 | 3.5 | G |
| 6,7-Dihydro-1,1,2,3,3,-pentamethyl-4(5H)-indanone ("CASHMERAN", trade name of IFF) | 5.0 | 4.5 | 4.5 | 4.0 | G |
| 4-acetyl-6-tert-butyl-1,2-dimethylindan ("CELESTOLIDE", trade name of International Flavors & Fragrances Inc.) | 5.0 | 2.0 | 2.5 | 3.5 | G |
| Cis-3-hexenyl anthranylate | 3.0 | 2.0 | 2.0 | 4.0 | G |
| 4-Hexenyl salicylate | 3.0 | 3.0 | 2.5 | 3.5 | G |
| Coumarin | 4.0 | 3.5 | 4.0 | 4.0 | G |
| Cyclopentadecanone | 5.0 | 2.5 | 2.5 | 4.0 | G |
| Dynascone | 4.0 | 4.5 | 5.0 | 3.5 | G |
| 3-Methyl-5-(2,2,3-trimethyl-3-cyclopenten-1-yl)-4-penten-2-o 1 ("EVANOL", trade name of Givaudan) | 4.0 | 4.0 | 4.0 | 4.0 | G |
| Ethyl salicylate | 3.5 | 3.5 | 1.5 | 4.0 | G |
| Eugenol acetate | 3.0 | 3.0 | 3.0 | 3.5 | G |
| Geranyl cyclopentanone | 5.0 | 3.5 | 3.5 | 4.0 | G |
| Oxacyclohexadecen-2-one ("HABANOLIDE", trade name of Firmenich) | 2.5 | 2.0 | 2.5 | 4.0 | G |
| Heliotropine | 4.5 | 4.0 | 4.0 | 4.0 | G |
| Heliotropyl acetone | 4.0 | 2.5 | 2.0 | 4.0 | G |
| Hexadecanolide | 2.5 | 2.0 | 2.5 | 4.0 | G |
| α-Ionone | 4.5 | 4.0 | 4.0 | 4.0 | G |
| β-Ionone | 4.5 | 3.0 | 2.5 | 4.5 | G |
| 7-Acetyl-1,2,3,4,5,6,7,8-octahydro-1,1,6,7-tetramethylnaphth alene ("ISO E SUPER", trade name of IFF) | 4.5 | 3.0 | 3.5 | 5.0 | G |

**Table 13**

| Names of fragrant materials | Stability | Scent from bottle mouth | Scent from foam | Persistence of scent | Ingredient |
|---|---|---|---|---|---|
| Isobutyl anthranylate | 3.0 | 2.0 | 2.5 | 4.0 | G |
| Isoeugenol acetate | 3.0 | 2.5 | 2.0 | 3.5 | G |
| δ-Decalactone | 4.0 | 3.5 | 3.5 | 4.5 | G |
| α-Methylionone | 4.5 | 3.0 | 3.0 | 4.0 | G |
| γ-Methylionone | 4.5 | 3.5 | 3.5 | 4.5 | G |
| 3-Methylcyclopentadecenone ("MUSCENONE DELTA", trade name of Firmenich) | 5.0 | 3.5 | 4.0 | 4.5 | G |
| 3-Methylcyclopentadecanone (muscone) | 4.0 | 2.5 | 3.0 | 4.0 | G |
| Ethylene dodecanedioate ("MUSK C-14", trade name of Takasago) | 2.5 | 2.0 | 2.0 | 4.0 | G |
| Musk ketone | 5.0 | 4.5 | 4.5 | 5.0 | G |
| 11-Oxa-16-hexadecanolide ("MUSK R-1", trade name of Quest) | 2.5 | 3.0 | 3.5 | 4.0 | G |
| 5-Cyclohexadecen-1-one ("MUSK TM-II", trade name of Soda) | 4.0 | 2.5 | 2.5 | 3.5 | G |
| Butyl anthranylate | 3.0 | 2.0 | 2.5 | 4.0 | G |
| Hexyl salicylate | 3.0 | 2.0 | 2.5 | 4.0 | G |
| Amyl salicylate | 3.5 | 2.5 | 2.0 | 4.0 | G |
| 10-Oxa-16-hexadecanolide ("OXALIDE", trade name of Takasago) | 2.5 | 2.0 | 2.0 | 3.5 | G |
| 4,6,6,7,8,8-Hexamethyl-1,3,4,6,7,8-hexahydrocyciopentabenz.op yran ("PEARLIDE PURE", trade name of Kao) | 4.0 | 3.0 | 3.0 | 5.0 | G |
| Cyclopentadecanolide ("PENTALIDE", trade name of Soda) | 2.5 | 2.0 | 2.0 | 4.0 | G |
| 2-Cyclohexylidene-2-phenyl acetonitrile ("PEONILE", trade name of Givaudan) | 4.5 | 3.5 | 3.5 | 4.5 | G |
| 3,3-Dimethyl-5-(2,2,3-trimethyl-3-cyclopenten-1-yl)-4-penten -2-ol ("POLYSANTOL", trade name of Firmenich) | 4.0 | 3.0 | 3.5 | 4.0 | G |
| 4-(Parahydroxyphenyl)-2-butanone ("RASPBERRY KETONE", trade name of Takasago) | 4.5 | 4.5 | 4.5 | 4.5 | G |
| 2-Methyl-4-(2,2,3-trimethyl-3-cyclopenten-1-yl)-2-buten-1-ol ("SANDALMYSORE CORE", trade name of Kao) | 4.5 | 3.5 | 4.0 | 4.0 | G |
| 3-Methyl-5-(2,2,3-trimethyl-3-cyclopenten-1-yl)-pentan-2-ol ("SANDALORE", trade name of Givaudan) | 4.0 | 2.5 | 3.0 | 4.0 | G |
| Cis-8-cyclohexadecenolide ("SCENTOLIDE", trade name of Synarom) | 4.0 | 2.0 | 2.0 | 4.0 | G |
| 6-Acetyl-1,1,2,4,4,7-hexatetralin ("TONALIDE", trade name of PFW) | 5.0 | 2.5 | 3.5 | 5.0 | G |

### Example F

The formula of a fragrance composition for an acidic shampoo will be shown.

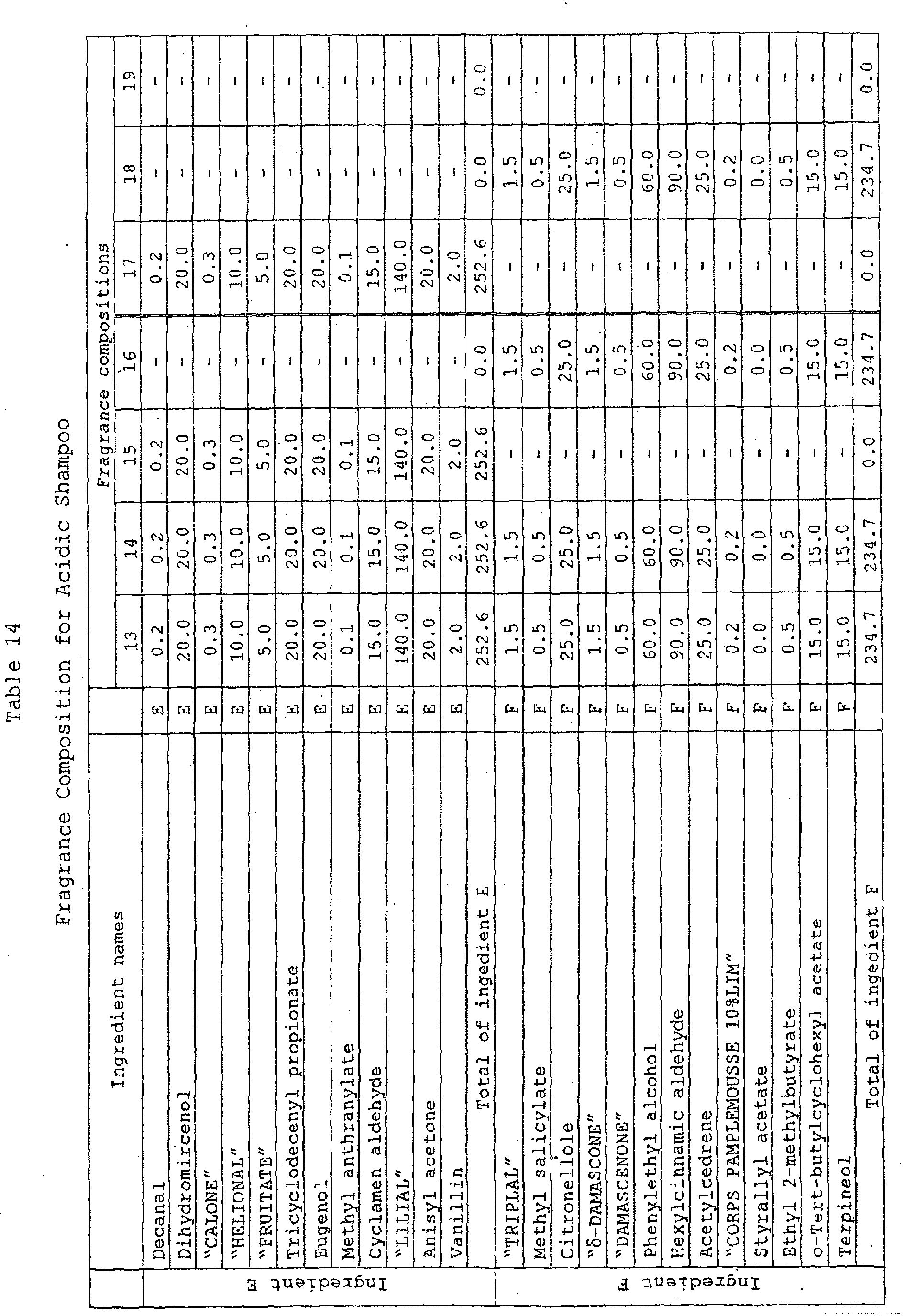

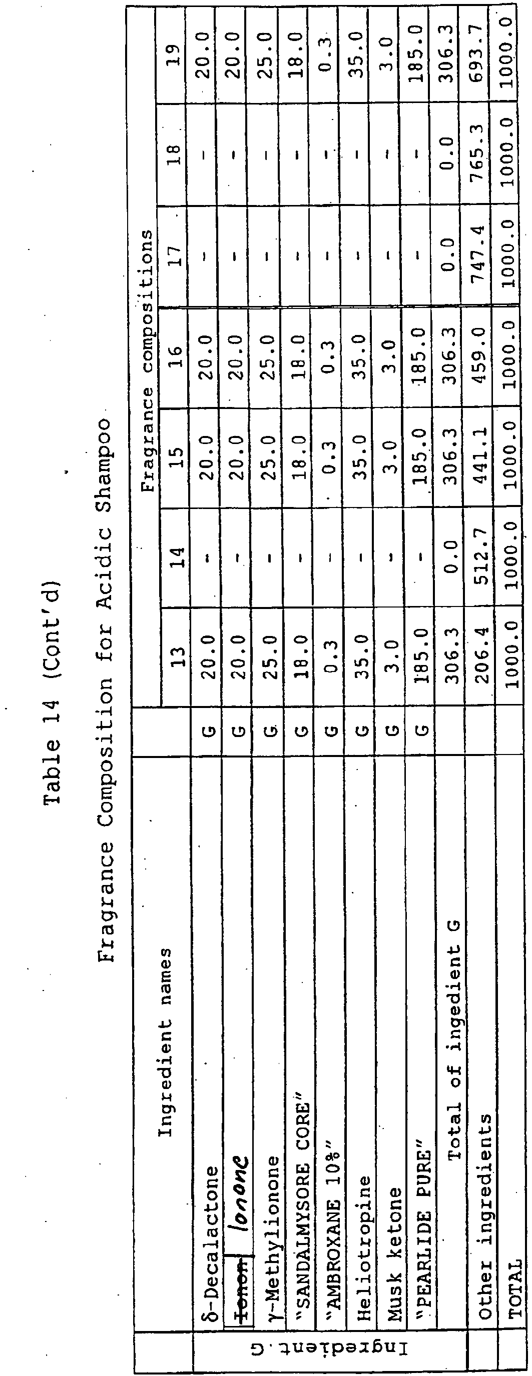

The fragrance compositions 13 to 19 shown in Table 14 were added at 0.5% to aliquots of the acidic shampoo base of Table 1 to fragrance the same. The thus-formulated shampoos were then ranked. The ranking results are shown in Table 15.

**Table 15**

| Acidic Shampoo | | | | | | | |
|---|---|---|---|---|---|---|---|
| | Examples | | | | Comparative Examples | | |
| | 9 | 10* | 11* | 12* | 3 | 4 | 5 |
| Composition of Table 1 | 99.50% | 99.50% | 99.50% | 99.50% | 99.50% | 99.50% | 99.50% |
| Fragrance composition 13 | 0.50% | | | | | | |
| Fragrance composition 14 | | 0.50% | | | | | |
| Fragrance composition 15 | | | 0.50% | | | | |
| Fragrance composition 16 | | | | 0.50% | | | |
| Fragrance composition 17 | | | | | 0.50% | | |
| Fragrance composition 18 | | | | | | 0.50% | |
| Fragrance composition 19 | | | | | | | 0.50% |
| TOTAL | 100.0% | 100.0% | 100.0% | 100.0% | 100.0% | 100.0% | 100.0% |
| | | | | | | | |
| Ranking of scent | | | | | | | |
| - Scent from foam | 5.0 | 4.0 | 4.0 | 3.0 | 3.5 | 2.5 | 2.0 |
| - Scent from bottle mouth | 5.0 | 4.0 | 3.5 | 4.0 | 2.0 | 3.0 | 3.0 |
| - Persistence of scent | 5.0 | 3.0 | 4.5 | 4.0 | 2.0 | 2.5 | 3.0 |
| Overall ranking of scent | 5.0 | 4.0 | 4.0 | 3.5 | 2.0 | 2.0 | 2.5 |
| Changes in scent with time (50°C/l month) | 5.0 | 4.0 | 5.0 | 4.0 | 4.0 | 4.0 | 4.0 |

| | | | | | | | |
|---|---|---|---|---|---|---|---|
| * Reference Example | | | | | | | |

### <Results>

Example 9 making the combined use of all three types of ingredients was found to give off a good scent in all stages, to keep the acid smell of the cosmetic base masked, and to have an excellent fragrance as a final product. Reference Examples 10 to 12 each making the combined use of two types of ingredients from the ingredients (E), (F) and (G) were each found in masking and balance to give off a good scent in all the stages, to keep the acid smell of the cosmetic base masked, and to have an excellent fragrance as a final product. In contrast, each of Comparative Examples 3 to 5 which made the use of only one type of ingredient (E), (F) or (G) was not balanced well in scent in each of the stages, and even by an overall judgement, was found to have an insufficient fragrance as a fragrance of a final product.

### <Overall Ranking>

A standard for the ranking item overall ranking of fragrance" in Examples F and G will now be shown.

The "overall ranking of fragrance" is a ranking determined by taking into comprehensive consideration the three ranking items of a scent from foam, a scent from a bottle mouth and the persistence of a scent, and the following ranking standard is relied upon.
5: A well-balanced scent is given off very well in all the stages, and the smell of a cosmetic base is completely masked.
4: A well-balanced scent is given off very well in all the stages, and the smell of a cosmetic base is masked well.
3: A well-balanced scent is given off very well in all the stages, and the smell of a cosmetic base is substantially masked.
2: A scent is poorly given off in at least one of the stages, and the masking of the smell of a cosmetic base is insufficient.
1: A scent is poorly given off in all the stages, and the masking of the smell of a cosmetic base is insufficient.

### Example G

The formula of a fragrance composition for an acidic shampoo will be shown.

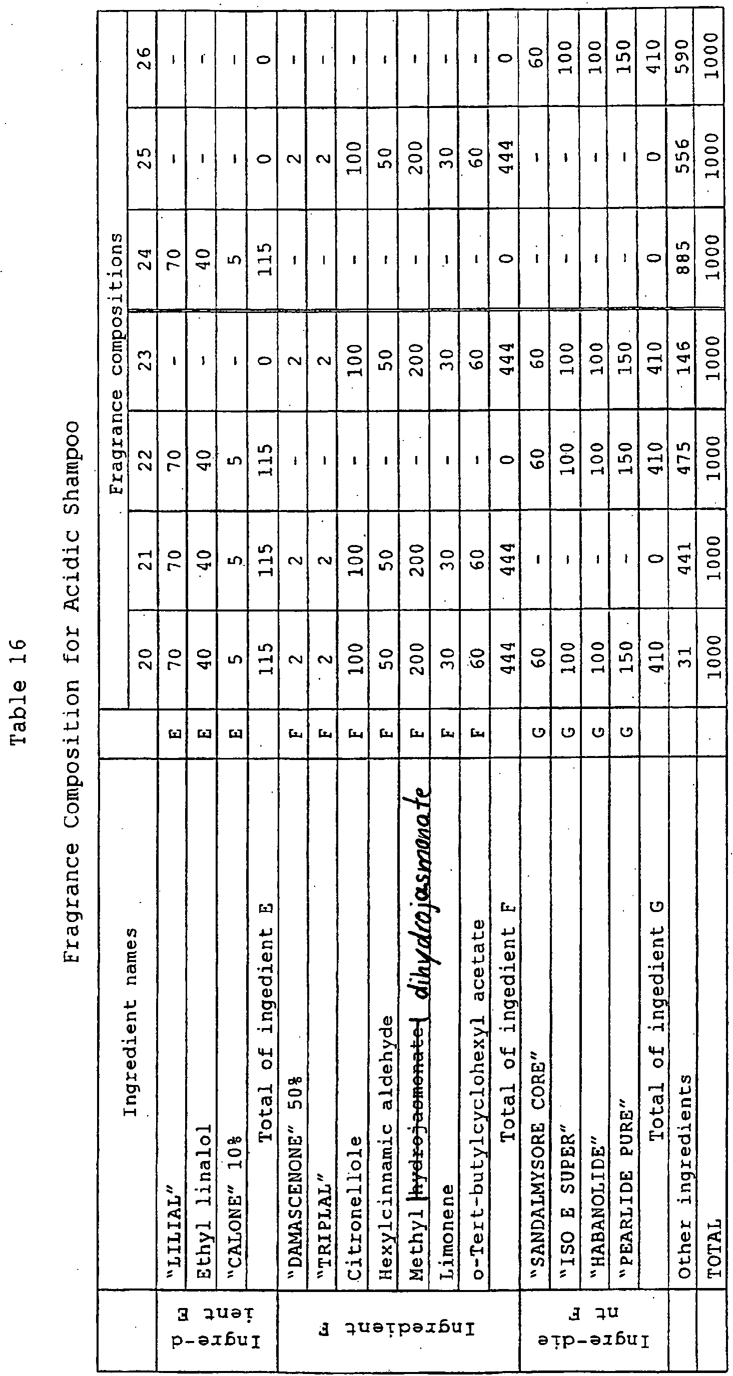

The fragrance compositions 20 to 26 shown in Table 16 were added at 0.5% to aliquots of the acidic shampoo base of Table 1 to fragrance the same. The thus-formulated shampoos were then ranked. The ranking results are shown in Table 17.

**Table 17**

| Acidic Shampoo | | | | | | | |
|---|---|---|---|---|---|---|---|
| | Examples | | | | Comparative Examples | | |
| | 13 | 14* | 15* | 16* | 6 | 7 | 8 |
| Composition of Table 1 | 99.50% | 99.50% | 99.50% | 99.50% | 99.50% | 99.50% | 99.50% |
| Fragrance composition 20 | 0.50% | | | | | | |
| Fragrance composition 21 | | 0.50% | | | | | |
| Fragrance composition 22 | | | 0.50% | | | | |
| Fragrance composition 23 | | | | 0.50% | | | |
| Fragrance composition 24 | | | | | 0.50% | | |
| Fragrance composition 25 | | | | | | 0.50% | |
| Fragrance composition 26 | | | | | | | 0.50% |
| TOTAL | 100.0% | 100.0% | 100.0% | 100.0% | 100.0% | 100.0% | 100.0% |
| | | | | | | | |
| Ranking of scent | | | | | | | |
| - Scent from foam | 5.0 | 4.0 | 4.0 | 3.0 | 3.0 | 2.5 | 2.0 |
| - Scent from bottle mouth | 5.0 | 4.0 | 3.5 | 4.0 | 3.0 | 3.0 | 2.0 |
| - Persistence of scent | 5.0 | 3.0 | 4.5 | 4.0 | 2.0 | 2.0 | 3.0 |
| Overall ranking of scent | 5.0 | 4.0 | 4.0 | 3.5 | 2.0 | 2.5 | 2.5 |
| Changes in scent with time (50°C/1 month) | 5.0 | 4.0 | 5.0 | 5.0 | 4.0 | 4.0 | 4.0 |

| | | | | | | | |
|---|---|---|---|---|---|---|---|
| * Reference Example | | | | | | | |

### <Results>

Example 13 making the combined use of all the three types of ingredients was found to give off a good scent in all the stages, to keep the acid smell of the cosmetic base masked, and to have an excellent fragrance as a final product. Reference Examples 14 to 16 each making the combined use of two types of ingredients from the ingredients (E), (F) and (G) were each found in masking and balance to give off a good scent in all the stages, to keep the acid smell of the cosmetic base masked, and to have an excellent fragrance as a final product. In contrast, each of Comparative Examples 6 to 8 which made the use of only one type of ingredient (E), (F) or (G) was not balanced well in scent in each of the stages, and even by an overall judgement, was found to have an insufficient fragrance as a fragrance of a final product.

### Industrial Applicability

The fragrance compositions used according to the present invention are excellent inmasking effects for a smell peculiar to hair cosmetic compositions having a pH of 1 to 5, and are also superb in long-term stability under high-temperature conditions. Acidic hair cosmetic compositions with the fragrance compositions added therein can each retain a good scent over a long period.

A well-balanced addition of the ingredients (E) to (G) to a fragrance composition makes it possible to obtain a fragrance composition for a hair cosmetic composition having a pH of 1 to 5, which can stably retain a good scent in the hair cosmetic composition having a pH of 1 to 5, gives off a good scent upon application of the hair cosmetic composition, is excellent in the persistence of scent after the application, and moreover, gives off a good scent in a bottled state (from a bottle mouth).

## Claims

1. A hair cosmetic composition having a pH of 1 to 5, which comprises the following ingredients (I) to (IV):
(I) from 1 to 50 wt.% of a surfactant;
(II) from 0.05 to 10 wt.% of oil;
(III) from 0.05 to 10 wt.% of an organic acid; and
(IV) a fragrance composition comprising the following ingredients (E), (F) and (G), wherein a content of said ingredient (E) is from 10 wt.% to 70 wt.% of said fragrance composition, a content of said ingredient (F) is from 5 wt.% to 60 wt.% of said fragrance composition, and a content of said ingredient (G) is from 10 wt.% to 60 wt.% of said fragrance composition:
(E) at least one ingredient selected from α-amylcynnamic aldehyde, acetophenone, decanal, undecanal, undecenal, dodecanal, 2-methylundecanal, octanal, nonanal, allylcyclohexyl propionate, allyl phenoxyacetate, anisaldehyde, anisyl acetate, anisyl acetone, borneol, 3-(p-tert-butylphenyl)-propanal, 7-methyl-3,5-dihydro-2H-benzodioxepin-3-one, cinnamyl acetate, cis-4-decenal, cis-jasmone, citronellyloxy acetaldehyde, dodecanenitrile, 2-methyl-3-(isopropylphenyl)-propanal (cyclamen aldehyde), dihydrojasmone, methyl dihydrojasmonate, dihydromircenol, tricyclodecylidene-4-butanal, ethyl 2-cyclohexylpropionate, ethyl 2,6,6-trimethyl-1,3-cyclohexadiene-1-carboxylate, ethyl isovalerate, ethyl linalol, ethyl vanillin, eugenol, 6-ethylidenoctahydro-5,8-methano-1H-1-benzopyran-2-one, ethyl tricyclodecan-2-yl-carboxylate, franeol, ethyl-3-(bicyclo[2.2.1]hepto-5-en-2-yl)-3-methyl glycidate, 2-methyl-3-(3,4-methylenedioxyphenyl)-propanol, cis-3-hexenyl methylcarbonate, 2-methyl-3-(4-tert-butylphenyl)-propanal, 4(3)-(4-hydroxy-4-methylpentyl)-3-cyclohexene-1-carboxaldeh yde, methyl anthranilate, methyl β-naphthyl ketone, 4(3)-(4-methyl-3-pentenyl)-3-cyclohexene-1-carboxaldehyde, 2-(2-(4-methyl-3-cyclohexen-1-yl)propyl)cyclopentanone, nookkatone, phenylacetaldehyde, 2-cyclohexylpropanal, 8-methoxytricyclodecane-4-carboxaldehyde, thymol, trans-2-decenal, trans-2-hexanal, tricyclodecenyl acetate, tricyclodecenyl propionate, 1-acezyl-2,6,10-trimethylcyclododeca-2,5,9-triene, and vanillin;
(F) at least one ingredient selected from 1-octen-3-yl acetate, 2,5-decadienal, 2,4-octadienal, 2,6-nonadienal, 1,3-oxathiane 2-methyl-4-propionate, 2-methylbutyl 2-methylbutyrate, 2-methylbutyl 2-isovalerate, 2-methylbutyl valerate, acetophenone, acetylcedrene [ethanone, 1-(2,3,4,7,8,8a-hexahydro-3,6,8,8-tetramethyl-1H-3a,7-metha noazulen-5-yl)-]β-methylnaphthyl ketone, α-damascon, hexanol, hexanal, heptanal, allylionone, allyl heptanoate, allyl hexanoate, α-pinene, α-terpinene, β-damascon, benzaldehyde, benzyl acetate, benzyl propionate, camphor, 2-methyl-3-(para-methoxyphenyl)-propylaldehyde, cedrene, 3-proylbicyclo(2.2.1)hept-5-ene-2-carbaldehyde, cinnamic alcohol, cis-3-hexenol, cis-3-hexenyl propionate, cis-jasmone, citral, citronellal, citronellol, citronellyl nitrile, thiol addition product of limonene, thiol substitution product of limonene, methyl cyclopentylidenacetate, dimethlbenzylcarbinyl acetate, 1-(2,6,6-trimethyl-1,3-cyclohexadien-1-yl)-2-buten-1-one, 1-(2,6,6-trimethyl-3-cyclohexen-1-yl)-2-buten-1-one, dihydrojasmone, ethyl maltol, ethyl trimethylhexanoate , ethyl 2-methylbutylate, ethyl butyrate, ethyl heptanoate, ethyl hexanoate, ethyl nonanoate, ethyl octanoate, ethyl propionate, ethyl valerate, fenchone, p-ethyl-2,2-dimethylhydrocinnamaldehyde, ethyl 2-tert-butylcyclohexylcarbonate, 2-sec-butylcyclohexanone, ethyl-2-methyl-1,3-dioxolan-2-acetate, geraniol, geranyl nitrile, 3-methyl-5-prop-2-cyclohexen-1-one, hexylcinnamic aldehyde, heptyl cyclopentanone, iron, isobutyl salicylate, 1-(2,4,4-trimethyl-2-cyclohexen-1-yl)-2-buten-1-one, L-carvone, 3,7-dimethyl-2(3),6-nonadienenitrile, limonene, L-menthol, menthone, maltol, 3-methyl-5-phenylpentanal, methyl dihydrojasmonate, methyl benzoate, methyl butyrate, methyl geranylate, methyl octanoate, methyl salicylate, methyl valerate, butyl butyrate, hexyl acetate, hexylcyclopentanone, amyl valerate, amyl butyrate, amyl propionate, orthomethoxycinnamic aldehyde, o-tert-butylcyclohexyl acetate, para-amylcyclohexanone, paracymene, phenylethyl alcohol, phenylethyl propionate, phenylhexanol, phenylpropyl acetate, 4-methyltricyclo[6.2.1.02.7]undecan-5-one, p-menthane-8-thiol-3-one, p-tert-butylcyclohexyl acetate, styrallyl acetate, terpineol, terpinolene, terpinyl propionate, trans-2-hexanal, trans-2-hexenyl acetate, trimethylhexanal, 2,4-dimethyl-3-cyclohexenylcarboxaldehyde, 2,2,5-trimethyl-5-pentylcyclopentanone, and 4-cyclohexyl-4-methyl-2-pentanone,
(G) allyl anthranillate, 1-(2-tert-butylcyclohexyloxy)-2-butanol, 7-cyclohexadecenolide (ambrettolide), (3α,6,6,9α-tetramethyldodecahydronaphtho[2.1-b]furan, 2-ethyl-4-(2,2,3-trimethyl-3-cyclopenten-1-yl)-2-buten-1-ol, benzophenone, benzyl alcohol, caryophyllene, 6,7-dihydro-1,1,2,3,3,-pentamethyl-4(5H)-indanone, 4-acetyl-6-tert-butyl-1,1-dimethylindan, cis-3-hexenyl anthranylate, 4-hexenyl salicylate, coumarin, cyclopentadecanone, dynascone, 3-methyl-5-(2,2,3-trimethyl-3-cyclopenten-1-yl)-4-penten-2-ol, ethyl salicylate, eugenol acetate, geranyl cyclopentanone, oxacyclohexadecen-2-one, heliotropine, heliotropyl acetone, hexadecanolide, α-ionone, β-ionone, 7-acetyl-1,2,3,4,5,6,7,8-octahydro-1,1,6,7-tetramethyl naphthalene, isobutyl anthranylate, isoeugenol acetate, δ-decalactone, α-methylionone, γ-methylionone, 3-methylcyclopentadecenone, 3-methylcyclopentadecanone (muscone), ethylene dodecanedioate, musk ketone, 11-oxa-16-hexadecanolide, 5-cyclohexadecen-1-one, butyl anthranylate, hexyl salicylate, amyl salicylate, 10-oxa-16-hexadecanolide, 4,6,6,7,8,8-hexamethyl-1,3,4,6,7,8-hexahydrocyclopenta benzopyran, 6-acetyl-1,1,2,4,4,7-hexatetralin, cis-8-cyclohexadecenolide, 3-methyl-5-(2,2,3-trimethyl-3-cyclopenten-1-yl)-pentan-2-ol, 2-methyl-4-(2,2,3-trimethyl-3-cyclopenten-1-yl)-2-buten-1-o 1-2-butanone, 3,3-dimethyl-5-(2,2,3-trimethyl-3-cyclopenten-1-yl)-4-pente n-2-ol, 2-cyclohexylidene-2-phenyl acetonitrile, and cyclopentadecanolide.
**characterized in that**
(IV) the fragrance composition comprises the following ingredients (E), (F) and (G), wherein a content of said ingredient (E) is from 10 wt.% to 70 wt.% of said fragrance composition, a content of said ingredient (F) is from 10 wt.% to 60 wt.% of said fragrance composition, and a content of said ingredient (G) is from 10 wt.% to 60 wt.% of said fragrance composition.
(E) at least one ingredient selected from decanal, undecanal, undecenal, dodecanal, 2-methylundecanal, 3-(p-tert-butylphenyl)-propanal, citronellyloxy acetaldehyde, 2-methyl-3-(isopropylphenyl)-propanal (cyclamen aldehyde), dihydrojasmone, dihydromircenol, tricyclodecylidene-4-butanal, ethyl vanillin, 6-ethylideneoctahydro-5,8-methano-2H-1-benzopyran-2-one, ethyl tricyclodecan-2-yl-carboxylate, 2-methyl-3-(3,4-methylenedioxyphenyl)-propanol, 4(3)-(4-hydroxy-4-methylpentyl)-3-cyclohexene-1-carboxaldeh yde, 4(3)-(4-methyl-3-pentenyl)-3-cyclohexene-1-carboxaldehyde, 2-(2-(4-methyl-3-cyclohexen-1-yl)propyl)cyclopentanone, 8-methoxytricyclodecane-4-carboxaldehyde,
(F) at least one ingredient selected from 1-octen-3-yl acetate, 2,4-octadienal, 2-methylbutyl 2-methylbutyrate, 2-methylbutyl 2-isovalerate, acetophenone, hexanol, hexanal, heptanal, allylionone, allyl hexanoate, α-terpinene, benzaldehyde, benzyl acetate, benzyl propionate, camphor, cedrene, 3-proylbicyclo(2.2.1)hept-5-ene-2-carbaldehyde, cinnamic alcohol, cis-3-hexenyl propionate, citral, citronellal, citronellyl nitrile, thiol addition product of limonene, 1-(2,6,6-trimethyl-1,3-cyclohexadien-1-yl)-2-buten-1-one, ethyl maltol, ethyl 2-methylbutylate, ethyl butyrate, ethyl hexanoate, ethyl valerate, fenchone, p-ethyl-2,2-dimethylhydrocinnamaldehyde, ethyl 2 -tert-butylcyclohexylcarbonate, 1-(2,4,4-trimethyl-2-cyclohexen-1-yl)-2-buten-1-one, L-carvone, menthone, maltol, 3-methyl-5-phenylpentanal, methyl benzoate, methyl butyrate, methyl octanoate, methyl salicylate, methyl valerate, hexyl acetate, amyl butyrate, amyl propionate, phenylethyl propionate, p-menthane-B-thiol-3-one, styrallyl acetate, trans-2-hexanal, trimethylhexanal, 2,4-dimethyl,-3-cyclohexenylcarboxaldehyde, and 4-cyclohexyl-4-methyl-2-pentanone,
(G) allyl anthranillate, 1-(2-tert-butylcyclohexyloxy)-2-butanol, (3α,6,6,9α-tetramethyldodecahydronaphtho[2.1-b]furan, 2-ethyl-4-(2,2,3-trimethyl-3-cyclopenten-1-yl)-2-buten-1-ol, benzophenone, cis-3-hexenyl anthranylate, 4-hexenyl salicylate, cyclopentadecanone, ethyl salicylate, geranyl cyclopentanone, heliotropyl acetone, β-ionone, 7-acetyl-1,2,3,4,5,6,7,8-octahydro-1,1,6,7-tetramethyl naphthalene, isobutyl anthranylate, δ-decalactone, 3-methylcyclopentadecenone, 3-methylcyclopentadecanone (muscone), musk ketone, butyl butyl anthranylate, amyl salicylate, 4,6,6,7,8,8-hexamethyl-1,3,4,6,7,8-hexahydrocyclopenta benzopyran, 6-acetyl-1,1,2,4,4,7-hexatetralin, cis-8-cyclohexadecenolide, 3-methyl-5-(2,2,3-trimethyl-3-cyclopenten-1-yl)-pentan-2-ol, 3,3-dimethyl-5-(2,2,3-trimethyl₋3-cyclopenten-1-yl)-4-pente n-2-ol, 2-cyclohexylidene-2-phenyl acetonitrile.

2. The hair cosmetic composition according to Claim 1, further comprising from 0.01 to 20 wt.% of an aromatic alcohol.

3. The hair cosmetic composition according to Claim 1 or 2, wherein the organic acid is an α-hydroxycarboxylic acid.

4. The hair cosmetic composition according to Claims 1-3, wherein the composition has a pH of 1-4.

5. The hair cosmetic composition according to any one of claims 1 to 4, wherein the surfactant is from 8 to 50 wt.%.

6. The hair cosmetic composition according to any one of claims 1 to 5, wherein the surfactant is an anionic surfactant.

7. The hair cosmetic composition according to any one of claims 1 to 6, wherein the oil is a silicone.

8. The hair cosmetic composition according to claim 7, wherein the silicone is selected from the group consisting of dimethylpolysiloxanes, amino-modified silicones, polyether-modified silicones, methylphenylpolysiloxane, fatty-acid-modified silicones, fluorine-modified silicones, cyclic silicones and alkyl-modified silicones.

9. The hair cosmetic composition according to any one of claims 1 to 8, further comprising a cationic polymer.

10. The hair cosmetic composition according to any one of claims 3 to 9, the α-hydroxycarboxylic acid is lactic acid and/or malic acid.

11. The hair cosmetic composition according to any one of claims 2 to 10, wherein the aromatic alcohol is benzyl alcohol, benzyloxyethanol or phenoxyethanol.

12. The hair cosmetic composition according to any one of claims 2 to 11, wherein the aromatic alcohol is from 0.1 to 10 wt.%.

13. The hair cosmetic composition according to claims 1 to 12, wherein the oil is from 0.1 to 5 wt.%.

## Patentansprüche

1. Haarkosmetik-Zusammensetzung mit einem pH-Wert von 1 bis 5, die die nachstehenden Bestandteile (I) bis (IV) umfasst:
(I) 1 bis 50 Gew.% eines Tensids;
(II) 0,05 bis 10 Gew.% eines Öls;
(III) 0,05 bis 10 Gew.% einer organischen Säure; und
(IV) eine Duftstoff-Zusammensetzung, die die nachstehenden Bestandteile (E), (F) und (G) umfasst, wobei der Gehalt an Bestandteil (E) 10 bis 70 Gew.% der Duftstoff-Zusammensetzung beträgt, der Gehalt an Bestandteil (F) 5 bis 60 Gew.% der Duftstoff-Zusammensetzung beträgt und der Gehalt an Bestandteil (G) 10 bis 60 Gew.% der Duftstoff-Zusammensetzung beträgt:
(E) mindestens ein Bestandteil, ausgewählt aus α-Amylzimtaldehyd, Acetophenon, Decanal, Undecanal, Undecenal, Dodecanal, 2-Methylundecanal, Octanal, Nonanal, Allylcyclohexylpropionat, Allylphenoxyacetat, Anisaldehyd, Anisylacetat, Anisylaceton, Borneol, 3-(p-tert-Butylphenyl)-propanal, 7-Methyl-3,5-dihydro-2H-benzodioxepin-3-on, Cinnamylacetat, cis-4-Decenal, cis-Jasmon, Citronellyloxyacetaldehyd, Dodecannitril, 2-Methyl-3-(isopropylphenyl)-propanal (Cyclamenaldehyd), Dihydrojasmon, Methyldihydrojasmonat, Dihydromircenol, Tricyclodecyliden-4-butanal, Ethyl-2-cyclohexylpropionat, Ethyl-2,6,6-trimethyl-1,3-cyclohexadien-1-carboxylat, Ethylisovalerat, Ethyllinalol, Ethylvanillin, Eugenol, 6-Ethylidenoctahydro-5,8-methano-1H-1-benzopyran-2-on, Ethyltricyclodecan-2-yl-carboxylat, Franeol, Ethyl-3-(bicyclo[2.2.1]hepto-5-en-2-yl)-3-methylglycidat, 2-Methyl-3-(3,4-methylendioxyphenyl)-propanol, cis-3-Hexenylmethylcarbonat, 2-Methyl-3-(4-tert-butylphenyl)-propanal, 4(3)-(4-Hydroxy-4-methylpentyl)-3-cyclohexen-1-carboxaldehyd, Methylanthranilat, Methyl-β-naphthylketon, 4(3)-(4-Methyl-3-pentenyl)-3-cyclohexen-1-carboxaldehyd, 2-(2-(4-Methyl-3-cyclohexen-1-yl)propyl)cyclopentanon, Nookkaton, Phenylacetaldehyd, 2-Cyclohexylpropanal, 8-Methoxytricyclodecan-4-carboxaldehyd, Thymol, trans-2-Decenal, trans-2-Hexanal, Tricyclodecenylacetat, Tricyclodecenylpropionat, 1-Acetyl-2,6,10-trimethylcyclododeca-2,5,9-trien und Vanillin,
(F) mindestens ein Bestandteil, ausgewählt aus 1-Octen-3-ylacetat, 2,5-Decadienal, 2,4-Octadienal, 2,6-Nonadienal, 1,3-Oxathian-2-methyl-4-propionat, 2-Methylbutyl-2-methylbutyrat, 2-Methylbutyl-2-isovalerat, 2-Methylbutylvalerat, Acetophenon, Acetylcedren [Ethanon, 1-(2,3,4,7,8,8a-Hexahydro-3,6,8,8-tetramethyl-1H-3a,7-methanoazulen-5-yl)-]-β-methylnaphthylketon, α-Damascon, Hexanol, Hexanal, Heptanal, Allylionon, Allylheptanoat, Allylhexanoat, α-Pinen, α-Terpinen, β-Damascon, Benzaldehyd, Benzylacetat, Benzylpropionat, Kampfer, 2-Methyl-3-(para-methoxyphenyl)-propylaldehyd, Cedren, 3-Proylbicyclo(2.2.1)-hept-5-en-2-carbaldehyd, Zimtalkohol, cis-3-Hexenol, cis-3-Hexenylpropionat, cis-Jasmon, Citral, Citronellal, Citronellol, Citronellylnitril, Thiol-Additionsprodukt von Limonen, Thiol-Substitutionsprodukt von Limonen, Methylcyclopentylidenacetat, Dimethlbenzylcarbinylacetat, 1-(2,6,6-Trimethyl-1,3-cyclohexadien-1-yl)-2-buten-1-on, 1-(2,6,6-Trimethyl-3-cyclohexen-1-yl)-2-buten-1-on, Dihydrojasmon, Ethylmaltol, Ethyltrimethylhexanoat, Ethyl-2-methylbutylat, Ethylbutyrat, Ethylheptanoat, Ethylhexanoat, Ethylnonanoat, Ethyloctanoat, Ethylpropionat, Ethylvalerat, Fenchon, p-Ethyl-2,2-dimethylhydrocinnamaldehyd, Ethyl-2-tert-butylcyclohexylcarbonat, 2-sek-Butylcyclohexanon, Ethyl-2-methyl-1,3-dioxolan-2-acetat, Geraniol, Geranylnitril, 3-Methyl-5-prop-2-cyclohexen-1-on, Hexylzimtsäurealdehyd, Heptylcyclopentanon, Iron, Isobutylsalicylat, 1-(2,4,4-Trimethyl-2-cyclohexen-1-yl)-2-buten-1-on, L-Carvon, 3,7-Dimethyl-2(3),6-nonadiennitril, Limonen, L-Menthol, Menthon, Maltol, 3-Methyl-5-phenylpentanal, Methyldihydrojasmonat, Methylbenzoat, Methylbutyrat, Methylgeranylat, Methyloctanoat, Methylsalicylat, Methylvalerat, Butylbutyrat, Hexylacetat, Hexylcyclopentanon, Amylvalerat, Amylbutyrat, Amylpropionat, Orthomethoxyzimtsäurealdehyd, o-tert-Butylcyclohexylacetat, para-Amylcyclohexanon, Paracymen, Phenylethylalkohol, Phenylethylpropionat, Phenylhexanol, Phenylpropylacetat, 4-Methyltricyclo[6.2.1.02.7]undecan-5-on, p-Menthan-8-thiol-3-on, p-tert-Butylcyclohexylacetat, Styrallylacetat, Terpineol, Terpinolen, Terpinylpropionat, trans-2-Hexanal, trans-2-Hexenylacetat, Trimethylhexanal, 2,4-Dimethyl-3-cyclohexenylcarboxaldehyd, 2,2,5-Trimethyl-5-pentylcyclopentanon und 4-Cyclohexyl-4-methyl-2-pentanon,
(G) Allylanthranillat, 1-(2-tert-Butylcyclohexyloxy)-2-butanol, 7-Cyclohexadecenolid (Ambrettolid), (3α,6,6,9α-Tetramethyldodecahydronaphtho[2.1-b]furan, 2-Ethyl-4-(2,2,3-trimethyl-3-cyclopenten-1-yl)-2-buten-1-ol, Benzophenon, Benzylalkohol, Caryophyllen, 6,7-Dihydro-1,1,2,3,3,-pentamethyl-4(5H)-indanon, 4-Acetyl-6-tert-butyl-1,1-dimethylindan, cis-3-Hexenylanthranylat, 4-Hexenylsalicylat, Cumarin, Cyclopentadecanon, Dynascon, 3-Methyl-5-(2,2,3-trimethyl-3-cyclopenten-1-yl)-4-penten-2-ol, Ethylsalicylat, Eugenolacetat, Geranylcyclopentanon, Oxacyclohexadecen-2-on, Heliotropin, Heliotropylaceton, Hexadecanolid, α-Ionon, β-Ionon, 7-Acetyl-1,2,3,4,5,6,7,8-octahydro-1,1,6,7-tetramethylnaphthalin, Isobutylanthranylat, Isoeugenolacetat, δ-Decalacton, α-Methylionon, γ-Methylionon, 3-Methylcyclopentadecenon, 3-Methylcyclopentadecanon (Muscon), Ethylendodecandioat, Moschus-Keton, 11-Oxa-16-hexadecanolid, 5-Cyclohexadecen-1-on, Butylanthranylat, Hexylsalicylat, Amylsalicylat, 10-Oxa-16-hexadecanolid, 4,6,6,7,8,8-Hexamethyl-1,3,4,6,7,8-hexahydrocyclopentabenzopyran, 6-Acetyl-1,1,2,4,4,7-hexatetralin, cis-8-Cyclohexadecenolid, 3-Methyl-5-(2,2,3-trimethyl-3-cyclopenten-1-yl)-pentan-2-ol, 2-Methyl-4-(2,2,3-trimethyl-3-cyclopenten-1-yl)-2-buten-1-ol-2-butanon, 3,3-Dimethyl-5-(2,2,3-trimethyl-3-cyclopenten-1-yl)-4-penten-2-ol, 2-Cyclohexyliden-2-phenylacetonitril und Cyclopentadecanolid,
**dadurch gekennzeichnet dass**,
(IV) die Duftstoff-Zusammensetzung, die nachstehenden Bestandteile (E), (F) und (G) umfasst, wobei der Gehalt an Bestandteil (E) 10 bis 70 Gew.% der Duftstoff-Zusammensetzung beträgt, der Gehalt an Bestandteil (F) 10 bis 60 Gew.% der Duftstoff-Zusammensetzung beträgt und der Gehalt an Bestandteil (G) 10 bis 60 Gew.% der Duftstoff-Zusammensetzung beträgt:
(E) mindestens ein Bestandteil, ausgewählt aus Decanal, Undecanal, Undecenal, Dodecanal, 2-Methylundecanal, 3-(p-tert-Butylphenyl)-propanal, Citronellyloxyacetaldehyd, 2-Methyl-3-(isopropylphenyl)-propanal (Cyclamenaldehyd), Dihydrojasmon, Dihydromircenol, Tricyclodecyliden-4-butanal, Ethylvanillin, 6-Ethylidenoctahydro-5,8-methano-2H-1-benzopyran-2-on, Ethyltricyclodecan-2-yl-carboxylat, 2-Methyl-3-(3,4-methylendioxyphenyl)-propanol, 4(3)-(4-Hydroxy-4-methylpentyl)-3-cyclohexen-1-carboxaldehyd, 4(3)-(4-Methyl-3-pentenyl)-3-cyclohexen-1-carboxaldehyd, 2-(2-(4-Methyl-3-cyclohexen-1-yl)propyl)cyclopentanon, 8-Methoxytricyclodecan-4-carboxaldehyd,
(F) mindestens ein Bestandteil, ausgewählt aus 1-Octen-3-ylacetat, 2,4-Octadienal, 2-Methylbutyl-2-methylbutyrat, 2-Methylbutyl-2-isovalerat, Acetophenon, Hexanol, Hexanal, Heptanal, Allylionon, Allylhexanoat, α-Terpinen, Benzaldehyd, Benzylacetat, Benzylpropionat, Kampfer, Cedren, 3-Proylbicyclo(2.2.1)hept-5-en-2-carbaldehyd, Zimtalkohol, cis-3-Hexenylpropionat, Citral, Citronellal, Citronellylnitril, Thiol-Additionsprodukt von Limonen, 1-(2,6,6-Trimethyl-1,3-cyclohexadien-1-yl)-2-buten-1-on, Ethylmaltol, Ethyl-2-methylbutylat, Ethylbutyrat, Ethylhexanoat, Ethylvalerat, Fenchon, p-Ethyl-2,2-dimethylhydrocinnamaldehyd, Ethyl-2-tert-butylcyclohexylcarbonat, 3-Methyl-5-prop-2-cyclohexen-1-on, 1-(2,4,4-Trimethyl-2-cyclohexen-1-yl)-2-buten-1-on, L-Carvon, Menthon, Maltol, 3-Methyl-5-phenylpentanal, Methylbenzoat, Methylbutyrat, Methyloctanoat, Methylsalicylat, Methylvalerat, Hexylacetat, Amylbutyrat, Amylpropionat, Paracymen, Phenylethylpropionat, p-Menthan-8-thiol-3-on, Styrallylacetat, trans-2-Hexanal, Trimethylhexanal, 2,4-Dimethyl-3-cyclohexenylcarboxaldehyd und 4-Cyclohexyl-4-methyl-2-pentanon,
(G) Allylanthranillat, 1-(2-tert-Butylcyclohexyloxy)-2-butanol, (3α,6,6,9α-Tetramethyldodecahydronaphtho[2.1-b]furan, 2-Ethyl-4-(2,2,3-trimethyl-3-cyclopenten-1-yl)-2-buten-1-ol, Benzophenon, cis-3-Hexenylanthranylat, 4-Hexenylsalicylat, Cyclopentadecanon, Ethylsalicylat, Geranylcyclopentanon, Heliotropylaceton, β-Ionon, 7-Acetyl-1,2,3,4,5,6,7,8-octahydro-1,1,6,7-tetramethylnaphthalin, Isobutylanthranylat, 8-Decalacton, 3-Methylcyclopentadecenon, 3-Methylcyclopentadecanon (Muscon), Moschus-Keton, Butylanthranylat, Amylsalicylat, 4,6,6,7,8,8-Hexamethyl-1,3,4,6,7,8-hexahydrocyclopentabenzopyran, 6-Acetyl-1,1,2,4,4,7-hexatetralin, cis-8-Cyclohexadecenolid, 3-Methyl-5-(2,2,3-trimethyl-3-cyclopenten-1-yl)-pentan-2-ol, 3,3-Dimethyl-5-(2,2,3-trimethyl-3-cyclopenten-1-yl)-4-penten-2-ol und 2-Cyclohexyliden-2-phenylacetonitril.

2. Haarkosmetik-Zusammensetzung gemäss Anspruch 1, die ferner 0,01 bis 20 Gew.% eines aromatischen Alkohols umfasst.

3. Haarkosmetik-Zusammensetzung gemäss Anspruch 1 oder 2, wobei die organische Säure α-Hydroxycarbonsäure ist.

4. Haarkosmetik-Zusammensetzung gemäss Ansprüchen 1 bis 3, wobei die Zusammensetzung einen pH-Wert von 1 bis 4 hat.

5. Haarkosmetik-Zusammensetzung gemäss irgendeinem der Ansprüche 1 bis 4, wobei das Tensid 8 bis 50 Gew.% beträgt.

6. Haarkosmetik-Zusammensetzung gemäss irgendeinem der Ansprüche 1 bis 5, wobei das Tensid ein anionisches Tensid ist.

7. Haarkosmetik-Zusammensetzung gemäss irgendeinem der Ansprüche 1 bis 6, wobei das Öl Silicon ist.

8. Haarkosmetik-Zusammensetzung gemäss Anspruch 7, wobei das Silicon aus der Gruppe bestehend aus Dimethylpolysiloxanen, Amino-modifizierten Siliconen, Polyether-modifizierten Siliconen, Methylphenylpolysiloxan, Fettsäure-modifizierten Siliconen, Fluor-modifizierten Siliconen, cyclischen Siliconen und Alkyl-modifizierten Siliconen ausgewählt ist.

9. Haarkosmetik-Zusammensetzung gemäss irgendeinem der Ansprüche 1 bis 8, die ferner ein kationisches Polymer umfasst.

10. Haarkosmetik-Zusammensetzung gemäss irgendeinem der Ansprüche 3 bis 9, wobei die α-Hydroxycarbonsäure Milchsäure und/oder Äpfelsäure ist.

11. Haarkosmetik-Zusammensetzung gemäss irgendeinem der Ansprüche 2 bis 10, wobei der aromatische Alkohol Benzylalkohol, Benzyloxyethanol oder Phenoxyethanol ist.

12. Haarkosmetik-Zusammensetzung gemäss irgendeinem der Ansprüche 2 bis 11, wobei der aromatische Alkohol 0,1 bis 10 Gew.% beträgt.

13. Haarkosmetik-Zusammensetzung gemäss den Ansprüchen 1 bis 12, wobei das Öl 0,1 bis 5 Gew.% beträgt.

## Revendications

1. Composition cosmétique pour les cheveux ayant un pH de 1 à 5, qui comprend les ingrédients (I) à (IV) suivants :
(I) de 1 à 50% en poids d'un agent tensioactif ;
(II) de 0,05 à 10% en poids d'huile ;
(III) de 0,05 à 10% en poids d'un acide organique ; et
(IV) une composition de fragrance comprenant les ingrédients (E), (F) et (G) suivants, dans laquelle une teneur dudit ingrédient (E) est de 10% en poids à 70% en poids de ladite composition de fragrance, une teneur dudit ingrédient (F) est de 5% en poids à 60% en poids de ladite composition de fragrance, et une teneur dudit ingrédient (G) est de 10% en poids à 60% en poids de ladite composition de fragrance :
(E) au moins un ingrédient choisi parmi l'aldéhyde α-amylcynnamique, l'acétophénone, le décanal, l'undécanal, l'undécénal, le dodécanal, le 2-méthylundécanal, l'octanal, le nonanal, le propionate d'allylcyclohexyle, le phénoxyacétate d'allyle, l'anisaldéhyde, l'acétate d'anisyle, l'anisylacétone, le bornéol, le 3-(p-tert-butylphényl)-propanal, la 7-méthyl-3,5-dihydro-2H-benzodioxépin-3-one, l'acétate de cinnamyle, le cis-4-décénal, la cis-jasmone, le citronellyloxyacétaldéhyde, le dodécanenitrile, le 2-méthyl-3-(isopropylphényl)-propanal (aldéhyde de cyclamen), la dihydrojasmone, le dihydrojasmonate de méthyle, le dihydromircénol, le tricyclodécylidène-4-butanal, le 2-cyclohexylpropionate d'éthyle, le 2,6,6-triméthyl-1,3-cyclohexadiène-1-carboxylate d'éthyle, l'isovalérate d'éthyle, l'éthyllinalol, l'éthylvanilline, l'eugénol, la 6-éthylidèneoctahydro-5,8-méthano-1H-1-benzopyran-2-one, le tricyclodécan-2-yl-carboxylate d'éthyle, le franéol, le glycidate d'éthyl-3-(bicyclo[2.2.1]hepto-5-èn-2-yl)-3-méthyle, le 2-méthyl-3-(3,4-méthylènedioxyphényl)-propanol, le méthylcarbonate de cis-3-hexényle, le 2-méthyl-3-(4-tert-butylphényl)-propanal, le 4(3)-(4-hydroxy-4-méthylpentyl)-3-cyclohexène-1-carboxaldéhyde, l'anthranilate de méthyle, la méthyl-β-naphtylcétone, le 4(3)-(4-méthyl-3-pentényl)-3-cyclohexène-1-carboxaldéhyde, la 2-(2-(4-méthyl-3-cyclohexèn-1-yl)propyl)cyclopentanone, la nootkatone, le phénylacétaldéhyde, le 2-cyclohexylpropanal, le 8-méthoxytricyclodécane-4-carboxaldéhyde, le thymol, le trans-2-décénal, le trans-2-hexanal, l'acétate de tricyclodécényle, le propionate de tricyclodécényle, le 1-acétyl-2,6,10-triméthylcyclododéca-2,5,9-triène, et la vanilline ;
(F) au moins un ingrédient choisi parmi l'acétate de 1-octèn-3-yle, le 2,5-décadiénal, le 2,4-octadiénal, le 2,6-nonadiénal, le 2-méthyl-4-propionate de 1,3-oxathiane, le 2-méthylbutyrate de 2-méthylbutyle, le 2-isovalérate de 2-méthylbutyle, le valérate de 2-méthylbutyle, l'acétophénone, l'acétylcédrène [éthanone,1-(2,3,4,7,8,8a-hexahydro-3,6,8,8-tétraméthyl-1H-3a,7-méthanoazulèn-5-yl)-]β-méthylnaphtylcétone, l'α-damascone, l'hexanol, l'hexanal, l'heptanal, l'allylionone, l'heptanoate d'allyle, l'hexanoate d'allyle, l'α-pinène, l'α-terpinène, la β-damascone, le benzaldéhyde, l'acétate de benzyle, le propionate de benzyle, le camphre, le 2-méthyl-3-(paraméthoxyphényl)-propylaldéhyde, le cédrène, le 3-propylbicyclo(2.2.1)hept-5-ène-2-carbaldéhyde, l'alcool cinnamique, le cis-3-hexénol, le propionate de cis-3-hexényle, la cis-jasmone, le citral, le citronellal, le citronellol, le nitrile de citronellyle, un produit d'addition de thiol du limonène, un produit de substitution de thiol du limonène, le cyclopentylidèneacétate de méthyle, l'acétate de diméthylbenzylcarbinyle, la 1-(2,6,6-triméthyl-1,3-cyclohexadièn-1-yl)-2-butèn-1-one, la dihydrojasmone, l'éthylmaltol, le triméthylhexanoate d'éthyle, le 2-méthylbutylate d'éthyle, le butyrate d'éthyle, l'heptanoate d'éthyle, l'hexanoate d'éthyle, le nonanoate d'éthyle, l'octanoate d'éthyle, le propionate d'éthyle, le valérate d'éthyle, la fenchone, le p-éthyl-2,2-diméthylhydrocinnamaldéhyde, le 2-tert-butylcyclohexylcarbonate d'éthyle, la 2-sec-butylcyclohexanone, le 2-méthyl-1,3-dioxolan-2-acétate d'éthyle, le géraniol, le nitrile de géranyle, la 3-méthyl-5-prop-2-cyclohexèn-1-one, l'aldéhyde hexylcinnamique, l'heptylcyclopentanone, le fer, le salicylate d'isobutyle, la 1-(2,4,4-triméthyl-2-cyclohexèn-1-yl)-2-butèn-1-one, la L-carvone, le 3,7-diméthyl-2(3),6-nonadiènenitrile, le limonène, le L-menthol, la menthone, le maltol, le 3-méthyl-5-phénylpentanal, le dihydrojasmonate de méthyle, le benzoate de méthyle, le butyrate de méthyle, le géranylate de méthyle, l'octanoate de méthyle, le salicylate de méthyle, le valérate de méthyle, le butyrate de butyle, l'acétate d'hexyle, l'hexylcyclopentanone, le valérate d'amyle, le butyrate d'amyle, le propionate d'amyle, l'aldéhyde orthométhoxycinnamique, l'acétate de o-tert-butylcyclohexyle, la para-amylcyclohexanone, le paracymène, l'alcool phényléthylique, le propionate de phényléthyle, le phénylhexanol, l'acétate de phénylpropyle, la 4-méthyltricyclo[6.2.1.02.7]undécan-5-one, la p-menthane-8-thiol-3-one, l'acétate de p-tert-butylcyclohexyle, l'acétate de styrallyle, le terpinéol, le terpinolène, le propionate de terpinyle, le trans-2-hexanal, l'acétate de trans-2-hexényle, le triméthylhexanal, le 2,4-diméthyl-3-cyclohexénylcarboxaldéhyde, la 2,2,5-triméthyl-5-pentylcyclopentanone, et la 4-cyclohexyl-4-méthyl-2-pentanone,
(G) l'anthranillate d'allyle, le 1-(2-tert-butylcyclohexyloxy)-2-butanol, le 7-cyclohexadécénolide (ambrettolide), le (3α,6,6,9α-tétraméthyldodécahydronaphto[2.1-b]furane, le 2-éthyl-4-(2,2,3-triméthyl-3-cyclopentèn-1-yl)-2-butèn-1-ol, la benzophénone, l'alcool benzylique, le caryophyllène, la 6,7-dihydro-1,1,2,3,3-pentaméthyl-4(5H)-indanone, le 4-acétyl-6-tert-butyl-1,1-diméthylindane, l'anthranylate de cis-3-hexényle, le salicylate de 4-hexényle, la coumarine, la cyclopentadécanone, la dynascone, le 3-méthyl-5-(2,2,3-triméthyl-3-3-cyclopentèn-1-yl)-4-pentèn-2-ol, le salicylate d'éthyle, l'acétate d'eugénol, la géranylcyclopentanone, l'oxacyclohexadécèn-2-one, l'héliotropine, l'acétone d'héliotropyle, l'hexadécanolide, l'α-ionone, la β-ionone, le 7-acétyl-1,2,3,4,5,6,7,8-octahydro-1,1,6,7-tétraméthylnaphtalène, l'anthranylate d'isobutyle, l'acétate d'isoeugénol, la δ-décalactone, l'α-méthylionone, la y-méthylionone, la 3-méthylcyclopentadécénone, la 3-méthylcyclopentadécanone (muscone), le dodécanedioate d'éthylène, la musccétone, le 11-oxa-16-hexadécanolide, la 5-cyclohexadécèn-1-one, l'anthranylate de butyle, le salicylate d'hexyle, le salicylate d'amyle, le 10-oxa-16-hexadécanolide, le 4,6,6,7,8,8-hexaméthyl-1,3,4,6,7,8-hexahydrocyclopentabenzopyrane, la 6-acétyl-1,1,2,4,4,7-hexatétraline, le cis-8-cyclohexadécénolide, le 3-méthyl-5-(2,2,3-triméthyl-3-cyclopentèn-1-yl)-pentan-2-ol, la 2-méthyl-4-(2,2,3-triméthyl-3-cyclopentèn-1-yl)-2-butèn-1-ol-2-butanone, le 3,3-diméthyl-5-(2,2,3-triméthyl-3-cyclopentèn-1-yl)-4-pentèn-2-ol, l'acétonitrile de 2-cyclohexylidène-2-phényle, et le cyclopentadécanolide ;
**caractérisé en ce que**
(IV) la composition de fragrance comprend les ingrédients (E), (F) et (G) suivants, dans laquelle une teneur dudit ingrédient (E) est de 10% en poids à 70% en poids de ladite composition de fragrance, une teneur dudit ingrédient (F) est de 10% en poids à 60% en poids de ladite composition de fragrance, et une teneur dudit ingrédient (G) est de 10% en poids à 60% en poids de ladite composition de fragrance :
(E) au moins un ingrédient choisi parmi le décanal, l'undécanal, l'undécénal, le dodécanal, le 2-méthylundécanal, le 3-(p-tert-butylphényl)-propanal, le citronellyloxyacétaldéhyde, le 2-méthyl-3-(isopropylphényl)-propanal (aldéhyde de cyclamen), la dihydrojasmone, le dihydromircénol, le tricyclodécylidène-4-butanal, l'éthylvanilline, la 6-éthylidèneoctahydro-5,8-méthano-2H-1-benzopyran-2-one, le tricyclodécan-2-yl-carboxylate d'éthyle, le 2-méthyl-3-(3,4-méthylènedioxyphényl)-propanol, le 4(3)-(4-hydroxy-4-méthylpentyl)-3-cyclohexène-1-carboxaldéhyde, le 4(3)-(4-méthyl-3-pentényl)-3-cyclohexène-1-carboxaldéhyde, la 2-(2-(4-méthyl-3-cyclohexèn-1-yl)propyl)cyclopentanone, le 8-méthoxytricyclodécane-4-carboxaldéhyde ;
(F) au moins un ingrédient choisi parmi l'acétate de 1-octèn-3-yle, le 2,4-octadiénal, le 2-méthylbutyrate de 2-méthylbutyle, le 2-isovalérate de 2-méthylbutyle, l'acétophénone, l'hexanol, l'hexanal, l'heptanal, l'allylionone, l'hexanoate d'allyle, l'a-terpinène, le benzaldéhyde, l'acétate de benzyle, le propionate de benzyle, le camphre, le cédrène, / le 3-propylbicyclo(2.2.1)hept-5-ène-2-carbaldéhyde, l'alcool cinnamique, le propionate de cis-3-hexényle, le citral, le citronellal, le nitrile, de citronellyle, un produit d'addition de thiol du limonène, la 1-(2,6,6-triméthyl-1,3-cyclohexadièn-1-yl)-2-butèn-1-one, l'éthylmaltol, le 2-méthylbutylate d'éthyle, le butyrate d'éthyle, l'hexanoate d'éthyle, le valérate d'éthyle, la fenchone, le p-éthyl2,2-diméthylhydrocinnamaldéhyde, le 2-tert-butylcyclohexylcarbonate d'éthyle, la 3-méthyl-5-prop-2-cyclohexèn-1-one, la 1-(2,4,4-triméthyl-2-cyclohexèn-1-yl)-2-butèn-1-one, la L-carvone, la menthone, le maltol, le 3-méthyl-5-phénylpentanal, le benzoate de méthyle, le butyrate de méthyle, l'octanoate de méthyle, le salicylate de méthyle, le valérate de méthyle, l'acétate d'hexyle, le butyrate d'amyle, le propionate d'amyle, le paracymène, le propionate de phényléthyle, la p-menthane-8-thiol-3-one, l'acétate de styrallyle, le trans-2-hexanal, le triméthylhexanal, le 2,4-diméthyl-3-cyclohexénylcarboxaldéhyde, et la 4-cyclohexyl-4-méthyl-2-pentanone,
(G) l'anthranillate d'allyle, le 1-(2-tert-butylcyclohexyloxy)-2-butanol, le (3α,6,6,9α-tétraméthyldodécahydronaphto[2.1-b]furane, le 2-éthyl-4-(2,2,3-triméthyl-3-cyclopentèn-1-yl)-2-butèn-1-ol, la benzophénone, l'anthranylate de cis-3-hexényle, le salicylate de 4-hexényle, la cyclopentadécanone, le salicylate d'éthyle, la géranylcyclopentanone, l'acétone d'héliotropyle, la β-ionone, le 7-acétyl-1,2,3,4,5,6,7,8-octahydro-1,1,6,7-tétraméthylnaphtalène, l'anthranylate d'isobutyle, la δ-décalactone, la 3-méthylcyclopentadécénone, la 3-méthylcyclopentadécanone (muscone), la musccétone, l'anthranylate de butyle, le salicylate d'amyle, le 4,6,6,7,8,8-hexaméthyl-1,3,4,6,7,8-hexahydrocyclopentabenzopyrane, la 6-acétyl-1,1,2,4,4,7-hexatétraline, le cis-8-cyclohexadécénolide, le 3-méthyl-5-(2,2,3-triméthyl-3-cyclopentèn-1-yl)-pentan-2-ol, le 3,3-diméthyl-5-(2,2,3-triméthyl-3-cyclopentèn-1-yl)-4-pentèn-2-ol, l'acétonitrile de 2-cyclohexylidène-2-phényle.

2. Composition cosmétique pour les cheveux selon la revendication 1, comprenant en outre de 0,01 à 20% en poids d'un alcool aromatique.

3. Composition cosmétique pour les cheveux selon la revendication 1 ou 2, dans laquelle l'acide organique est un acide α-hydroxycarboxylique.

4. Composition cosmétique pour les cheveux selon les revendications 1 à 3, dans laquelle la composition a un pH de 1 à 4.

5. Composition cosmétique pour les cheveux selon l'une quelconque des revendications 1 à 4, dans laquelle l'agent tensioactif est de 8 à 50% en poids.

6. Composition cosmétique pour les cheveux selon l'une quelconque des revendications 1 à 5, dans laquelle l'agent tensioactif est un agent tensioactif anionique.

7. Composition cosmétique pour les cheveux selon l'une quelconque des revendications 1 à 6, dans laquelle l'huile est un silicone.

8. Composition cosmétique pour les cheveux selon la revendication 7, dans laquelle le silicone est choisi parmi le groupe consistant en des diméthylpolysiloxanes, des silicones modifiés avec un amino, des silicones modifiés avec un polyéther, un méthylphénylpolysiloxane, des silicones modifiés avec un acide gras, des silicones modifiés avec un fluor, des silicones cycliques et des silicones modifiés avec un alkyle.

9. Composition cosmétique pour les cheveux selon l'une quelconque des revendications 1 à 8, comprenant en outre un polymère cationique.

10. Composition cosmétique pour les cheveux selon l'une quelconque des revendications 3 à 9, dans laquelle l'acide α-hydroxycarboxylique est l'acide lactique et/ou l'acide malique.

11. Composition cosmétique pour les cheveux selon l'une quelconque des revendications 2 à 10, dans laquelle l'alcool aromatique est un alcool benzylique, un benzyloxyéthanol ou un phénoxyéthanol.

12. Composition cosmétique pour les cheveux selon l'une quelconque des revendications 2 à 11, dans laquelle l'alcool aromatique est de 0,1 à 10% en poids.

13. Composition cosmétique pour les cheveux selon les revendications 1 à 12, dans laquelle l'huile est de 0,1 à 5% en poids.
